(19) Europäisches Patentamt
European Patent Office
Office européen des brevets

(11) **EP 4 185 238 B1**

(12) **EUROPEAN PATENT SPECIFICATION**

(45) Date of publication and mention
of the grant of the patent:
**23.10.2024 Bulletin 2024/43**

(21) Application number: **21754718.1**

(22) Date of filing: **21.07.2021**

(51) International Patent Classification (IPC):
*A61F 2/00* (2006.01)      *A61F 2/12* (2006.01)
*B33Y 10/00* (2015.01)      *B33Y 80/00* (2015.01)

(52) Cooperative Patent Classification (CPC):
**A61F 2/0059; A61F 2/12; A61F 2/28; A61F 2/3094;
B33Y 10/00; B33Y 80/00;** A61F 2002/2835;
A61F 2002/30593; A61F 2002/30985

(86) International application number:
**PCT/EP2021/070353**

(87) International publication number:
**WO 2022/018124 (27.01.2022 Gazette 2022/04)**

(54) **A THREE-DIMENSIONAL IMPLANT FOR TISSUE RECONSTRUCTION**

EIN DREIDIMENSIONALES IMPLANTAT ZUR GEWEBEREKONSTRUKTION

UN IMPLANT TRIDIMENSIONNEL POUR LA RECONSTRUCTION DES TISSUS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.07.2020 EP 20186961**

(43) Date of publication of application:
**31.05.2023 Bulletin 2023/22**

(73) Proprietor: **BellaSeno GmbH
04103 Leipzig (DE)**

(72) Inventors:
• **KHANI, Navid
04107 Leipzig (DE)**
• **LUCAROTTI, Sara
04103 Leipzig (DE)**
• **CHHAYA, Mohit
04105 Leipzig (DE)**

(74) Representative: **Schiweck Weinzierl Koch
Patentanwälte Partnerschaft mbB
Ganghoferstraße 68 B
80339 München (DE)**

(56) References cited:
EP-A1- 2 995 278        WO-A1-2018/130949
WO-A1-2019/217335       WO-A1-2019/238716
US-A1- 2014 243 995     US-A1- 2014 255 647
US-A1- 2018 264 718     US-B1- 6 993 406

**Description**

**CROSS-REFERENCE TO RELATED APPLICATIONS**

The present application claims the right of priority of European patent application 20186961.7 filed with the European Patent Office on 21 July 2020.

**FIELD OF THE INVENTION**

The present description relates to the field of implants, and in particular to implants for insertion into a patient, and a method for forming an implant.

**BACKGROUND OF THE INVENTION**

The insertion of implants into patients is performed in surgeries worldwide. Depending on the required use of the implant, an implant may be required to meet certain criteria. Such criteria may relate to the geometry of the implant and/or the mechanical stability of the implant. In some instances, such as wherein an implant may be used in reconstructive surgery, the implant may have to be handled by a surgeon before, during and/or after the implant is inserted into a patient. It is desirable to have an implant which meets the geometrical and mechanical criteria required by the patient, and which is easily handled and/or manipulated by surgeons performing implant surgery.

European application no. EP 2 995 278 A1 discloses an implant for tissue reconstruction which comprises a scaffold structure that includes a void system for the generation of prevascularized connective tissue with void spaces for cell and/or tissue transplantation and a method of manufacturing such an implant, to the internal architecture of such an implant, to a removal tool for mechanical removal of space-occupying structures from such an implant, to a kit comprising such an implant and such a removal tool, to a removal device for the removal of superparamagnetic or fenomagnetic space-occupying structures from such an implant.

US patent application no. US 2014/0243995 A1 discloses a three-dimensional tissue engineering scaffold device and related methods are disclosed herein. The tissue engineering scaffold includes a plurality of polymers sheets. Each polymer sheet includes a plurality of micro-scale pores defined through the polymer sheet. The polymer sheets of the tissue engineering scaffold are aligned and stacked such that some of the pores of neighboring sheets are offset along at least one axis of the pores. The offset pores create features within the tissue engineering scaffold.

**SUMMARY**

Various embodiments relate to providing an implant which may be flexibly tailored to meet criteria required of a reconstruction to a patient's body part, and which also enhances and improves the handleability and manipulability of the implant by surgeons performing surgery.

The implant may be used for guiding a needle through the implant for implant surgery.

Various embodiments relate to a three-dimensional implant for tissue reconstruction or tissue augmentation for insertion into a patient. The implant comprises a plurality of planar layers. A first group of sublayers comprises a plurality of strands oriented in first direction. A second group of sublayers comprises a plurality of strands oriented in a second direction. The sublayers of the first group of sublayers and the sublayers of the second group of sublayers are arranged alternatingly in a third direction. The plurality of layers forming a three-dimensional structure comprising a plurality of hollow channels extending in the third direction, wherein the implant is compressible at least along the third direction. Each hollow channel comprises a first sidewall extending in the third direction and comprises a plurality of strand segments oriented in a first direction and a plurality of gaps arranged alternatingly, and a second sidewall extending in the third direction and comprising a plurality of strand segments oriented in the second direction and a plurality of gaps arranged alternatingly. At least one of the first sidewall and the second sidewall of the hollow channel is an undulating sidewall. The plurality of strand segments of the undulating sidewall belong to different layers of the implant. Adjacent strand segments of the undulating sidewall are separated by a gap. The adjacent strand segments have a lateral offset with respect to each other to create a pattern of a plurality of peaks and a plurality of troughs of the undulating sidewall.

Although not being part of the invention, further disclosed herein is a method of tissue reconstruction or tissue augmentation, wherein the method comprises implanting into the body of a subject an implant as defined herein.

**BRIEF DESCRIPTION OF THE DRAWINGS**

The foregoing and other features of the present disclosure will become more fully apparent from the following description and appended claims, taken in conjunction with the accompanying drawings. It is understood that the accompanying drawings depict only several embodiments in accordance with the present disclosure and are, therefore, not to be considered limiting of its scope. The disclosure will be described with additional specificity and detail through use of the accompanying drawings, such that the advantages of the present disclosure can be more readily ascertained, in which:

Figs. 1A and 1B show respectively perspective and cross-sectional side-view illustrations of a three-dimensional implant 100 for insertion into a patient;

Fig. 1C shows a gap of the implant between two adjacent strand segments of a sidewall;

Figs. 1D and 1E show respectively a first sublayer and a second sublayer of an implant;

Fig. 1F shows an illustration of a beam having two simple supports undergoing a deflection;

Fig. 1G shows an illustration of deflection in adjacent strand segments in response to insertion of a needle;

Fig. 1H shows adjacent strand segments arrangement with zero lateral offset;

Fig. 1I shows adjacent strand segments arrangement with a lateral offset greater than zero;

Figs. 2A and 2B show respectively perspective and cross-sectional side-view illustrations of a further implant for insertion into a patient;

Fig. 3A shows a perspective illustration of an implant suitable for use as breast implant;

Figs. 3B and 3C shows a perspective side view illustration and a perspective top view illustration of an implant including a plurality contouring strands and surface filler strands;

Figs. 3D shows perspective views of the implant;

Fig. 4A shows an implant without reversibly expandable gaps;

Fig. 4B shows an image of a cannula for fat injection being inserted into an implant with reversibly expandable gaps;

Fig. 4C shows an example of possible regions of insertions for multi-injections testing;

Fig. 4D shows stress-strain curves of strands of different materials;

Fig. 5A shows an implant after a fat-injection mock surgery;

Figs. 5B to 5D show respectively images of the implant after fat-injection;

Fig. 6 shows a flow chart of a method 600 for forming an implant;

Figs. 7A to 7G show implants with at least one undulating sidewall.

## DETAILED DESCRIPTION

In the following detailed description, reference is made to the accompanying drawings that show, by way of illustration, specific embodiments in which the claimed subject matter may be practiced. These embodiments are described in sufficient detail to enable those skilled in the art to practice the subject matter. It is to be understood that the various embodiments, although different, are not necessarily mutually exclusive. For example, a particular feature, structure, or characteristic described herein, in connection with one embodiment, may be implemented within other embodiments without departing from the spirit and scope of the claimed subject matter. References within this specification to "one embodiment" or "an embodiment" mean that a particular feature, structure, or characteristic described in connection with the embodiment is included in at least one implementation encompassed within the present description. Therefore, the use of the phrase "one embodiment" or "in an embodiment" does not necessarily refer to the same embodiment. In addition, it is to be understood that the location or arrangement of individual elements within each disclosed embodiment may be modified without departing from the spirit and scope of the claimed subject matter. The following detailed description is, therefore, not to be taken in a limiting sense, and the scope of the subject matter is defined only by the appended claims, appropriately interpreted, along with the full range of equivalents to which the appended claims are entitled. In the drawings, like numerals refer to the same or similar elements or functionality throughout the several views, and that elements depicted therein are not necessarily to scale with one another, rather individual elements may be enlarged or reduced in order to more easily comprehend the elements in the context of the present description.

The terms "over", "to", "between" and "on" as used herein may refer to a relative position of one layer with respect to other layers. One layer "over" or "on" another layer may be directly in contact with the other layer or may have one or more intervening layers. One layer "between" layers may be directly in contact with the layers or may have one or more intervening layers. The phrase "A, B and/or C" as used herein may mean "A", "B", "C", "A and B", B and C", "A and C", and "A and B and C".

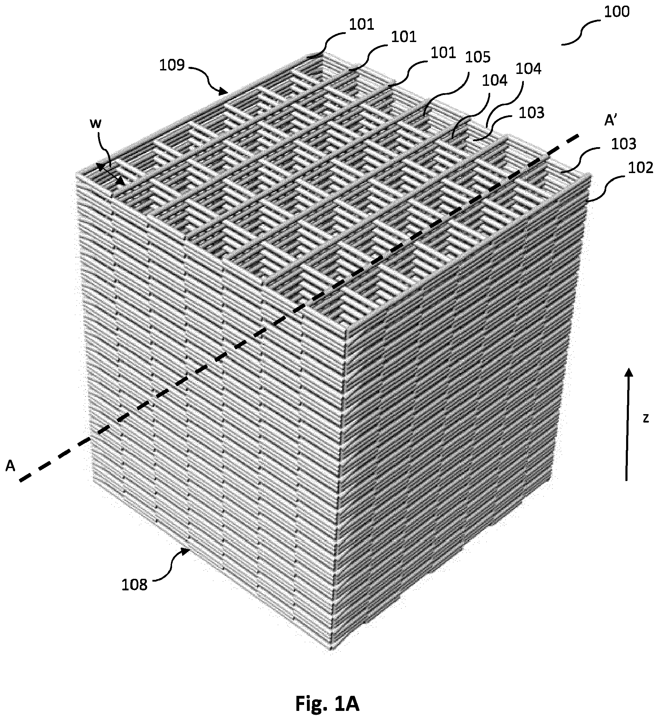

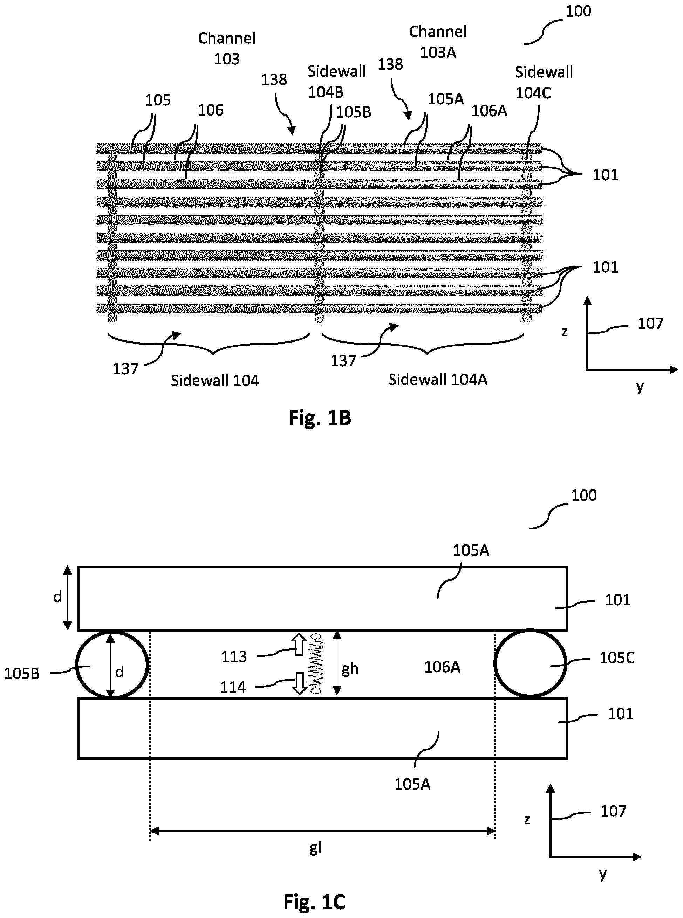

Figs. 1A and 1B show respectively perspective and cross-sectional side-view illustrations of an implant 100 for insertion into a patient.

As shown in Fig. 1A, the implant 100 comprises a plurality of strands 101 forming a three-dimensional structure 102. The three-dimensional structure 102 comprises a plurality of hollow channels 103. Each hollow channel 103 comprises a plurality of sidewalls 104. A sidewall 104 comprises a plurality of strand segments 105 and a plurality of gaps 106 (shown in Fig. 1B) arranged alternatingly so that a gap 106 is formed between adjacent strand segments 105 of the sidewall 104. The plurality of gaps 106 are reversibly expandable gaps.

Generally, a hollow channel 103 may be formed by at least three sidewalls contiguous with each other (or e.g. intersecting each other) so that a hollow space is enclosed by the sidewalls. In the example of Fig. 1A, a plurality of hollow channels 103, each having a square cross-section is shown. A (or each) hollow channel 103 with a square cross-section may have four intersecting sidewalls 104.

Fig. 1B shows a cross-sectional side view illustration of the implant 100 through the line A-A'. The side-view illustration shows a plurality of strands (also referred to as filaments) 101 of the three-dimensional structure 102, wherein the plurality of (parallel) strands 101 form sidewalls 104, 104A of two adjacent (e.g. directly adjacent) channels 103, 103A of the three-dimensional structure 102. As shown in Fig. 1B, and taking hollow channel 103A as an example, the hollow channel 103A may include a first sidewall 104A (shown as a sidewall parallel to the page) formed from the plurality of parallel strands 101. The hollow channel 103A my further include a second sidewall 104B (shown as strands going into and/or out of the page), a third sidewall 104C (shown as strands going into and/or out of the page) and a fourth sidewall (not shown). The third sidewall 104C may be an opposite facing sidewall to the second sidewall 104B. Both the second sidewall 104B and the third sidewall 104C may be contiguous to the first sidewall 104A and the fourth sidewall. The fourth sidewall may be an opposite facing sidewall to the first sidewall 104A. Where the sidewalls intersect, their strands may be cross-cross each other in an alternating fashion.

A (or each) sidewall 104 of a channel 103, such as the sidewall 104A of the hollow channel 103A may include a plurality of consecutive strand segments 105A arranged consecutively in the z-direction (vertical). Each strand segment 105A may be a portion (or segment) of a longer continuous strand 101 forming parts of other sidewalls 104 or channels 103. Optionally, the plurality of consecutive strand segments 105A forming the sidewall 104A of the hollow channel 103A may be substantially parallel to each other. Each sidewall 104A may further include a plurality of gaps 106A.

The plurality of gaps 106A and the plurality of strand segments 105A of the hollow channel 103A may be arranged alternatingly, such that a (e.g. one) gap 106A may be located between adjacent (e.g. directly consecutive) strand segments 105A of the sidewall 104A (e.g. between two successive strand segments 105A of the sidewall 104A). Adjacent (e.g. directly adjacent, or e.g. successive) strand segments may be separated by a gap 106A.

Fig. 1C shows a gap 106A between two adjacent strand segments 105A of a (one, single) sidewall 104A of the hollow channel 103A of the implant 300.

The gap 106A may also be referred to as a slit and/or a spacing, and refers to a blank space between the lengths of two adjacent strand segments 105A. The gap 106A may be defined by two pairs of intersecting strands 101 forming a perimeter of the gap 106A (e.g. bordering, or e.g. enclosing the gap 106A). The first pair of strands segments 105A (parallel to the y-direction) of the first sidewall 104A may be opposite facing strands and/or may optionally be substantially parallel to each other. The second pair of strand segments 105B, 105C may be opposite facing strands of the second sidewall 104B and the third sidewall 104C and may be substantially parallel to each other. The gap 106A may be the area enclosed by the two pairs of intersecting strands. The first pair of strand segments 105A forming the gap 106A may be adjacent strand segments 105A of the same (first) sidewall 104A of the hollow channel 103A. The second pair of strand segments may be opposite strands of the second sidewall 104B and the third sidewall 104C.

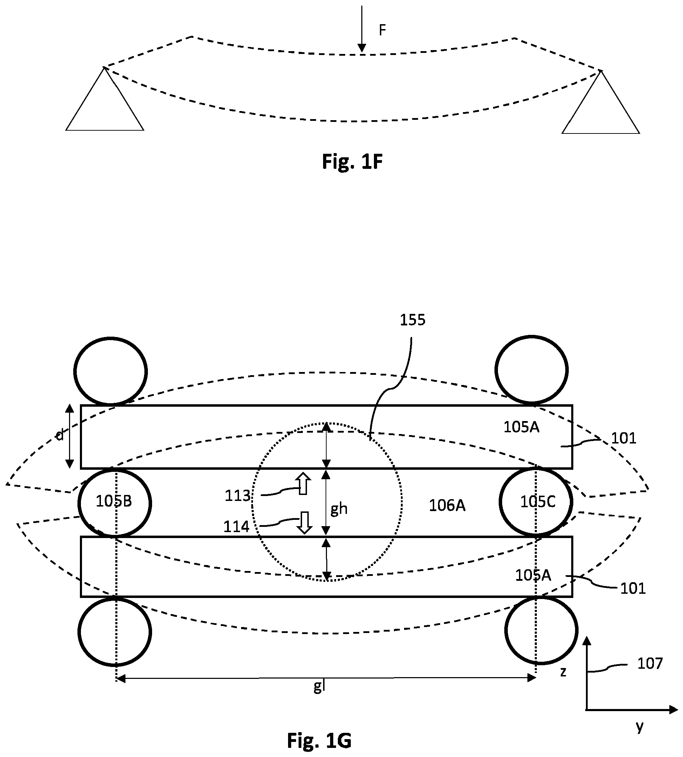

The gap 106A may have, or may be defined by a baseline gap height, h. The baseline (or resting) gap height, gh, may be a minimal (or smallest) height between the two adjacent strand 105A when the implant 100 is at rest. Additionally, or alternatively, the baseline gap height, gh, may be a height of the gap 106A at a mid-point region of the gap 106A (e.g. at a mid-point region of the strand segments 105A of the gap 106A. Additionally or alternatively, the baseline gap height may be an average (mean) gap height of more than 80 % of the gaps of the implant when the implant is at rest. Additionally, the gap 106A may have, or may be defined by a baseline gap length, gl, which is the maximal (or largest) dimension of the gap 106A. Additionally, the base line gap length, gl, may be the length of the strand segment between the second pair of strands 105B, 105C, such as between the two closest edges of the first strand 105B and the second strand 105C (Fig. 1C), or alternatively, from center of the first strand 105B to the center of the second strand 105C (Fig. 1G). The second pair of strands may be fused to the first pair of strand segments where the two pairs intersect, and the second pair of strands may form a simple support for the first pair of strand segments at opposite ends of the gap length, gl.

The two pairs of intersecting strands 101 may be fused or joined at the intersection areas. Additionally or optionally, opposite facing strand segments 105A may have some sag or droop so that the baseline gap height, gh, is equal to or less than the average strand diameter, d. When the gap height is expanded, (e.g. by inserting a needle through the gap) the shape of the gap 106 may be changed, or modified. Optionally, the area enclosed by the two pairs of intersecting strand segments 105A, 105B, 105C may remain unchanged. Alternatively or optionally, the area enclosed by the two pairs of intersecting strands 101 may increase to more than 110 % (or e.g. more than 120 %, or e.g. more than 150 %, or e.g. more than 200 %, or e.g. between 120 % and 250 %, or e.g. between 120 % and 200 %) of the originally enclosed area.

Fig. 1C also illustrates a spring-like character of a reversibly expandable gap 106 (such as gap 106A). If a tension force (indicated by the opposing arrows 113, 114) is applied to the opposite facing strand segments 105A of the gap 106A, the gap height, gh, increases. Additionally, each of the opposite facing strand segments 105A of the gap 106A may experience opposing tensional forces. When the tension forces are applied to the strand segments 105A of the gap 106A, the gap 106A may be configured to be expandable with respect to the baseline gap height, gh. For example, the reversibly expandable gap 106 may be expandable to more than 110 % (or e.g. more than 120 %, or e.g. more than 150 %, or e.g. more than 200 %, or e.g. between 110% and 250%, or e.g. between 120 % and 250 %, or e.g. between 120 % and 200 %) of the baseline gap height, gh and/or average strand diameter, d. The reversibly expandable gap 106A may recover or return to its original (resting) gap height after the tension forces (113, 114) are removed from the strands 105A of the gap 106A (even at the same ambient pressure and temperature). For example, the reversibly expandable gap 106 may be configured to recover or return to less than 110 % (or e.g. to less than 105 %, or e.g. to 100 %, or e.g. between 80 % and 115 %, or e.g. between 90 % and 110 %) of its original gap height, gh, and/or average strand diameter, d, after the tension forces exerted on the strands 105A has been removed.

Optionally, the baseline gap length, gl may be more than 2 times (or e.g. more than 5 times, or e.g. more than 10 times) larger than the baseline gap height, gh. Optionally, a baseline gap height, gh, may lie between 0.05 mm and 5 mm (or e.g. between 0.1 mm and 2 mm, or e.g. between 0.5 mm and 1.5 mm). Optionally, an average thickness of the plurality of strands 101 may lie between 0.05 mm and 5 mm (or e.g. between 0.1 mm and 2 mm, or e.g. between 0.5 mm and 1.5 mm). Optionally, the baseline gap length, gl may be more than 2 times (or e.g. more than 5 times, or e.g. more than 10 times) larger than the diameter, d, (or thickness) of the strands 101. For example, the baseline gap length, gl may be less than 25 mm (or e.g. between 0.5 mm and 25 mm). Optionally a baseline gap height, gh, may lie between 40 % to 100 % (or e.g. between 40 % and 80 %, or e.g. between 40 % and 60 %) of the average (mean) strand diameter, d, of the strands 101 of the implant. It may be understood that although Fig. 1C shows only one respective strand segment 105B, 105C of each of the second sidewall 104B and the third sidewall 104C, more than one strand of each of the second sidewall 104B and the third sidewall 104C may be arranged between the first pair of strand segments 105A of the first sidewall 104A. In which case, the baseline gap height, gh, may depend on the total thickness in the z-direction of the second pair of strands. For example, the baseline gap height, gh, may lie between 40 % to 100 % (or e.g. between 40 % and 80 %, or e.g. between 40 % and 60 %) of the total thickness in the z-direction of the second pair of strands.

As shown in Fig. 1B, each hollow channel 103, 103A may include at least the first sidewall 104A comprising a first plurality of consecutive strand segments 105A and a first plurality of reversibly expandable gaps 106. The hollow channel 103A may further include a second sidewall 104B comprising a second plurality of consecutive strand segments and a second plurality of reversibly expandable gaps. The second sidewall 104B may be contiguous to the first sidewall 104A. The strand segments 105A of the first plurality of consecutive strand segments 105A and strand segments 105B of the second plurality of consecutive strand segments 105B may be arranged alternatingly in a direction between a first end 137 of a longitudinal axis of the hollow channel 103A and second end 138 of the longitudinal axis of the hollow channel 103A.

The two sidewalls 104, 104A of the adjacent channels 103, 103A may be separated (or divided) by the further sidewall 104B. The further sidewall 104B may comprise a plurality of strands (shown in Fig. 1B to be going into the page). The strands of the further sidewall 104B may intersect the strands 101 forming the sidewalls 104A, 104A. The channels of the plurality of hollow channels 103 may be arranged adjacently (e.g. directly adjacently) to each other. The adjacent channels 103A, 103B may share the common sidewall 104B.

The implant 100 may have different sizes depending on the purpose of the implant. In the x-direction, the implant may have a dimension of up to 30 cm (or e.g. between 1 cm and 30 cm, or e.g. between 10 cm and 15 cm). In the y-direction, the implant may have a dimension of up to 30 cm (or e.g. between 1 cm and 30 cm, or e.g. between 10 cm and 15 cm). In the x-direction, the implant may have a dimension of up to 30 cm (or e.g. between 1 cm and 30 cm, or e.g. between 10 cm and 15 cm).

The plurality of strands 101 of the three-dimensional structure 102 of the implant 100 may constitute (or may make up) a material volume of the implant. The material volume occupied by the plurality of strands 101 may lie between 5 % and 70 % (or e.g. between 50 % and 70 %, or e.g. between 50 % and 60 %) of the total resting implant volume. Additionally, or optionally, the gaps 106 of the implant may constitute (or may include) between 30 % and 95 % (or e.g. between 30 % and 50 %, or e.g. between 40 % and 50 %) of the total resting implant volume. Of the material volume of the three-dimensional structure 102 of the implant, the plurality of sidewalls 104 of the implant may constitute (or may include) between 80 % to 100 % of the material volume. The rest of the material volume of the implant 100 which is not formed by the plurality of sidewalls 104 may be contributions from contouring lines and/or surface filler lines, for example. Optionally, the strand segments 105 of a sidewall 104 may constitute (or may make up) less than 90 % (or e.g. less than 50 %, or e.g. between 50 % and 70 %, or e.g. between 50 % and 60 %) of the sidewall 104, with the rest being of the sidewall 104 being occupied by the gaps 106. Optionally, at least 80 % (or e.g. at least 95 % or e.g. 100 %) of all the sidewalls 104 of the plurality of sidewalls 104 may include the reversibly expandable gaps 106. Additionally or optionally, at least 50 % (or e.g. at least 60 %, or e.g. at least 70 %) of all the gaps 106 of the implant 100 are reversibly expandable gaps.

As shown in Fig. 1A, the implant 100 may form (or may be) a three-dimensional structure 102, which may be a mesh-like structure or scaffold structure. For example, the plurality of strands 101 (or lines) may be configured to form the mesh-like three-dimensional structure 102. The three-dimensional structure 102 may define (or may have) the resting volume of the implant 100. The resting volume (cm³) of the implant 100 may be the volume of the implant 100 before inserting the implant 100 into the patient to construct and/or reconstruct soft tissue. The resting volume of the implant 100 may be the volume of the implant 100 at rest. An implant 100 at rest may be the state of the implant wherein only one outer surface (e.g. outer surface 108) of the implant 100 experience an external force, such as when the implant 100 is at rest on (or in contact with, or seated on) a carrier surface (e.g. a table surface, or e.g. a board). For example, an implant 100 at rest may mean that a first outer surface 108 of the implant may be in contact with the carrier surface, and a second (opposite facing) outer surface 109 may be free from any tensional and/or compressive forces. In other words, the resting volume of the implant 100 may be the volume of the implant 100 without opposing compressive or tensional forces acting on the surfaces of the implant. The resting volume of the implant 100 may be derived based on a construction volume (the desired or required volume) of the implant 100 to be inserted into the patient. For example, optionally, the implant 100 may be configured to be compressible to less than the construction volume, so that the implant 101 comprises or attains the construction volume after insertion into the patient.

The plurality of strands 101 may be a plurality of lines, or string-like material. A strand 101 (or line, or filament) may have a length and a cross-sectional diameter, d. The diameter of the strand 101 may be the average dimension of the strand 101, such as the smallest cross-sectional dimension of the strand 101. Optionally, the length of the strand 101 may be larger (e.g. at least 5 times larger, or e.g. at least 10 times larger, or e.g. at least 20 times larger) than the diameter of the strand 101. The strand diameter may optionally be between 300 $\mu$m and 350 $\mu$m, for example.

The term hollow channel 103 may refer to a channel wherein at least 70 % (or e.g. at least 80 %, or e.g. at least 90 %) of the channel volume enclosed by the sidewalls of that channel is unfilled or unoccupied by any material such as strand segments or strands. For example, the hollow channel 103 may not necessarily be limited to being a completely (100 %) unfilled channel.

The three-dimensional structure 102 may include a plurality of substantially planar layers (e.g. parallel to the x-y plane) arranged or stacked successively over (on top of) each other in the z-direction 107 (e.g. vertical direction). Optionally, each planar layer may include a plurality of strands 101 forming a two-dimensional lattice arrangement of unit cells. The individual layers of the implant 100 may be arranged successively (stacked and/or one above the other), such that the unit cells of successive layers (formed on top of each other) may form the plurality of hollow channels 103. Each channel 103 may be formed from (or may include) a column of unit cells from successive layers stacked on top of each other. For example, in the implant 100 shown in Figs. 1A to 1C, the implant 100 may include predominantly square-shaped unit cells of a plurality of layers arranged on top of each other to form hollow channels 103 having a square-shaped cross-section. Optionally, the unit cells may be repeated regularly throughout more than 50 % (or e.g. more than 80 %, or e.g. more than 90 %, or e.g. more than 95 %) of the resting volume of the three-dimensional lattice structure 102. The lattice structure 102 may thus include a plurality of adjacent (e.g. directly adjacent) unit cells, connected to each other throughout the lattice structure 102. A unit cell may be the smallest and most basic unit of the three-dimensional lattice structure 102.

Optionally, each planar layer of unit cells may include a first sublayer (or a first group of sublayers) including strands oriented in a first direction, and an adjacent second sublayer (or second group of sublayers) of strands oriented in a second direction different to the first direction.

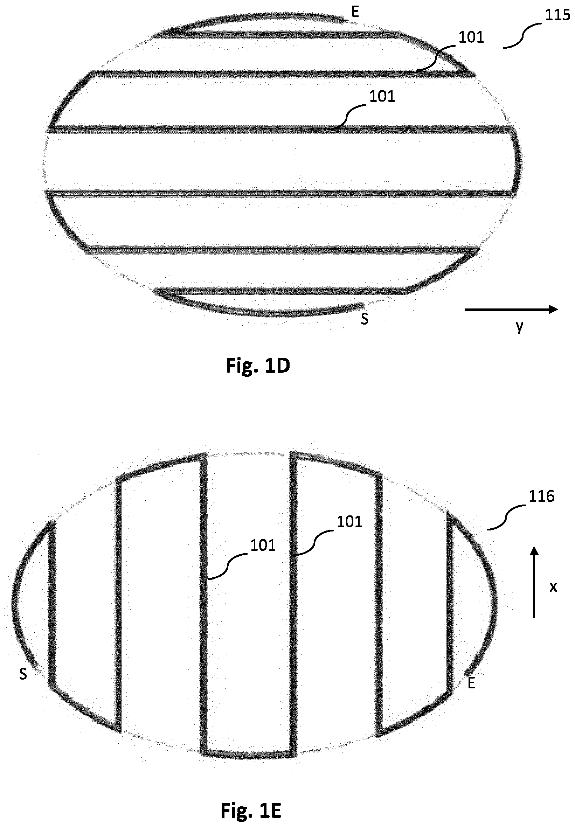

Figs. 1D show a first sublayer of an implant 100, the first sublayer 115 including strands 101 oriented in the first direction (e.g. a y-direction). Figs. 1E show a second sublayer 116 of an implant 100, the first sublayer 116 including strands 101 oriented in the second direction (e.g. a x-direction). Optionally, the strands 101 within each respective sublayer may be parallel to each other (e.g. an acute angle between the strands within a sublayer, or between the strand of best fit for sinusoidal strands, may lie within +/ - 5°). The plurality of strands 101 of the first sublayer 115 and the plurality of strands 101 of the second sublayer 116 may intersect at intersection points or intersection regions to form the two-dimensional lattice arrangement of a layer. Optionally, the strands 101 of (or e.g. within) each sublayer 115, 116, may be part of a continuous strand meandering continuously from a start point, S, of the sublayer to an end point, E, of the sublayer. For example, optionally, the strands of the first sublayer 115 may be part of a continuous sublayer strand meandering continuously from the start point, S, to the end point, E, of the first sublayer. Optionally, the strands of the second sublayer 116 may be part of a continuous sublayer strand meandering continuously from the start point, S, to the end point, E, of the second sublayer. A continuous strand of a sublayer may have a plurality of straight portions oriented in a first direction. A plurality of straight portions of a sublayer may be connected by meandering portions. The meandering portions may be formed at least partially on the boundary or perimeter of the layer, and may form surface filler lines or a sidewall of a channel at the surface of the implant. Each sublayer may have its own boundary or perimeter at which meandering portions (or surface filler lines are formed). Optionally, the strands may be straight strands, or alternatively, the strands may be sinusoidal zig-zag strands, wherein the unit cells may have a "free-form" shape.

The first sublayer 115 and the second sublayer 116 may be arranged such that their respective strands 101 intersect to form the two-dimensional lattice arrangement of unit cells. The intersecting strands 101 of the first sublayer 115 and the second sublayer 116 may be arranged, so that each unit cell formed from the intersecting strands may include or may be referred to as a pore, having a pore size. The intersecting strands may form or define a geometry (e.g. shape, dimension, pore size) of the individual unit cells. Each two-dimensional unit cell may have a pore size, defining the dimension of the unit cell. The pore size of the two-dimensional unit cell may be described in terms of its diameter, width and/or pore area, For example, the pore size, w, of the unit cell of a layer may be referred to as the diameter or width of the hollow channel 103 (as shown in Fig. 1A). Optionally, an average pore size of the plurality of unit cells of the three-dimensional structure 102 may be at least 0.5 mm (or e.g. at least 0.75 mm, or e.g. at least 0.8 mm, or e.g. at least 1 mm, or e.g. at least 1.5 mm, or e.g. at least 2 mm, or e.g. at least 5 mm). A pore area of at least 25 % (or e.g. at least 70 %, or e.g. at least 80 %) of the surface pores of the three-dimensional structure 101 may be at least 0.75 mm$^2$ (or e.g. at least 1 mm$^2$, or e.g. at least 3 mm$^2$). The surface pore area may be the area enclosed by intersecting strands defining a surface pore.

The plurality of two-dimensional unit cells may be polygonal-shaped, triangular-shaped unit cells, diamond-shaped unit cells, rhomboid-shaped unit cells, square-shaped unit cells, ellipsoidal shaped, sinusoidal shaped, and/or hexagonal shaped unit cells. It may be understood that the implant 100 may optionally include unit cells have predominantly (e.g. more than 50 %, or e.g. more than 60 %, or e.g. more than 70 %, or e.g. more than 80 %) one of those shapes throughout the volume of the implant 100, or alternatively, the implant 100 may include unit cells with a variety of different shapes. A sidewall 104 of the hollow channel 103 may be formed from the plurality of strands 101 lying substantially parallel to the x-y plane, vertically stacked in the z-direction (third direction), and oriented substantially in the same direction. The sidewalls 104 of a hollow channel 103 may be arranged such that a cross-section shape of the hollow channel 103 is one of the shapes from the following group of shapes: polygon, triangle, diamond, rhomboid, square, ellipsoid, and sinusoidal, and hexagonal.

In comparison to the pores, the gaps 106 of the implant 100 may refer to (or may be) the smallest spacings between any adjacent strands forming the hollow channel 103. For example, the gaps 106 may have the smallest area enclosed by strands of the implant (e.g. smallest gap area), compared to the pore size area of the unit cells. Optionally, a gap area of a gap 106 (which may the area enclosed by the two pairs of opposing strands defining the gap 106), may be less than 50 % (or e.g. less than 40 % or e.g. less than 30 %) of the pore size area.

Each hollow channel 103 may extend along a longitudinal axis of the hollow channel 103. The longitudinal axis may be a line including the mid-points of the sidewalls of the hollow channel 103. The hollow channels 103 may extend between the first outer surface region 108 and a second outer surface region 109 of the three-dimensional structure 102, for example. A channel 103 (e.g. optionally, each channel, or e.g. one or more channels 103) of the plurality of hollow channels 103 may be configured to extend from the first outer surface region 108 towards the second outer surface region 109. The exact positions and/or tilt angles of the plurality of hollow channels 103 may be configured according to the needs of the patient. Optionally, the plurality of hollow channels 103 may be parallel to each other (e.g. an acute angle between the sidewalls of adjacent channels may lie within +/ - 5°). Alternatively, the plurality of hollow channels 103 may converge towards a region (or point) of convergence, wherein the region of convergence is located outside the first outer surface region 108 or the second outer surface region 109. Optionally, the hollow channels 103 may include zig-zag channels, slanted channels and/or tapered channels. Optionally, the hollow channels 103 may be channels slanted with respect to the first outer surface region 108. For example, of the unit cells forming the column of unit cells, a unit cell of a second layer may have a lateral offset (in the x-direction or y-direction) with respect to a unit cell of an adjacent first layer. The lateral offset value between the first unit cell and the second unit cell may lie between 0 % and 50 % (or e.g. between 0 % and 20 %, or e.g. between 5 % and 10 %) of a pore size of the unit cell. Optionally, within the same column, a unit cell of each layer may have the same lateral offset with respect to a unit cell of a directly previous layer. Optionally, at least 80 % (or e.g. at least 70 %, or e.g. at least 50 %) of the unit cells of the same column (forming the same channel) may have the same pore size and the same pore shape. Alternatively, in the case of tapered channels, unit cells forming the same channel may have different pore sizes (e.g. the pore sizes of the unit cells may decrease or increase towards one of the outer surface regions).

The plurality of strands 101 may be configured such that the gap 106A is reversibly expandable when the implant is at rest and/or even when the implant 100 is under compression. When a (physical or mechanical) compression force is exerted on more than one outer surface of the implant 101, the implant 101 may be compressible to less than 80 % (or e.g. less than 70 %, or e.g. less than 60 %, or e.g. less than 50 %, or e.g. less than 30 %, or e.g. between 30 % and 95 %, or e.g. between 45 % and 80 %, or e.g. between 45 % and 70 %, or e.g. between 80 % and 95 %) of its resting volume.

The expandability of the gaps of the implant may be explained with reference to a deflection of a beam. The gaps of the implant may be expandable, meaning that the strands are able to deflect and/or bend, without breaking, in response to one or more forces exerted on the strand, such that the gap height between two adjacent strand segments increases. A bending stress $\sigma$ of a beam (or strand segment) undergoing simple bending in response to an applied external force may be expressed as

$$\sigma = \frac{MC}{I}$$

As shown in Fig. 1F, such a beam may be simply supported at both ends, and the bending moment may be a reaction induced in the beam when the external force or moment is applied to the beam. $\sigma$ may be the bending stress. M may be the bending moment, c may be the distance from the neutral axis. I may be the moment of inertia of the beam cross-section. The maximum bending moment M caused by a centrally applied load between the supports may be described or defined by the equation

$$(2): \quad M = \frac{FL}{4}$$

L may be the length of the beam and F may be the force exerted on the beam. A maximum bending stress, $\sigma_{max}$ may be represented by the equation

$$(1): \quad \sigma_{max} = \frac{MC_{max}}{I}$$

M may be the maximum bending moment, $c_{max}$ may be the maximum distance from the neutral axis. I may be the moment of inertia of the beam cross-section. Deflection, $\delta$, of a centre load on a beam with two simple supports may be described by

$$(3): \quad \delta = \frac{FL^3}{48EI}$$

L may be the length of the beam, F may be the force exerted, E may be the Young's Modulus and I may be the moment of inertia of the beam. When considering the implant 100, the deflection capability, $\delta$, of a strand of an implant may result in an expanding gap. The deflection capability may reflect or may be a desired deflection capacity of the strand, and/or a desired expandability of the gap height (e.g. how much a gap is expected to expand by).

Fig. 1G shows an illustration of the implant illustrated in Fig. 1C. As shown in Fig. 1G, the insertion of a needle 155 into the gap may exert one or more forces 113, 114 on the implant. The needle may have a diameter, nd, which may be larger than the baseline gap height, gh. The gap length, may be represented by gl, which may be the length of the strand segment between the center of the first strand 105B and the center of the second strand 105C. The first strand and second strand 105C may act as simple supports for the adjacent strand segments 101 of the gap.

The insertion of the needle results in deflection, $\delta$ in each of the two consecutive strand segments 105a. Each strand segment 105a may behave like a beam with two simple supports 105B, 105c undergoing a bending stress. A deflection of the adjacent strand segments 105A caused by the insertion of the needle may be represented by the dotted lines. The deflection may be represented by the equation

$$\delta = \frac{FL^3}{48EI}$$

The maximum bending stress of a strand which may be subject to a force, such as by insertion of the needle 155, may be

$$(4): \quad \sigma_{max} = \frac{MR}{I} = \frac{\frac{FL}{4} \times R}{I} = \frac{FLR}{4I}$$

R may be the radius of the strand (e.g. $d = 2 \times R$).

Using equations (3) and (4),

$$(5): \quad F = \frac{4I\sigma_{max}}{LR} = \frac{48EI\delta}{L^3},$$

and an equation for maximum stress, $\sigma_{max}$, may be obtained:

$$(6): \quad \sigma_{max} = \frac{12ER\delta}{L^2}$$

A rule may be applied for elastic deformation, such that the value of $\sigma_{max}$ does not exceed $\sigma_{yield}$, which is the yield strength of the material of the strand. Thus,

$$(7): \quad \frac{12ER\delta}{L^2} \le \sigma_{yield}$$

The deflection capability, $\delta$, may be represented by the equation

$$(8): \quad \delta = \frac{nd - gh}{2}$$

The reversibly expandable gap 106 may be expandable to between 110 % and 250 % of the baseline gap height, gh. This may occur, if a needle diameter, nd, is between 110 % and 250 % of the base line gap height, gh. These parameters may be expressed by the equation

$$1.1 \times gh \le 2\delta + gh \le 2.5 \times gh$$

and

$$(9): \quad 0.05 \times gh \le \delta \le 0.75 \times gh$$

E and $\sigma_{yield}$ are material properties of the strand material of the implant 100. R, the radius of the strand, and gl, the length of the strand segment between the two supports 105B, 105C are geometric features of the strands of the implant 100. Equation (10) may be obtained based on equation (7).

$$(10): \quad \frac{R}{gl^2} \le \frac{\sigma_{yield}}{12E\delta}$$

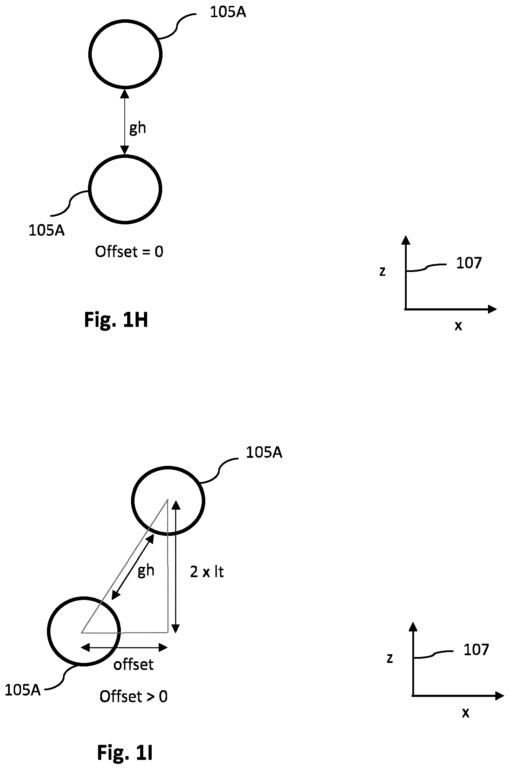

Fig. 1H shows an illustration of the adjacent strands 105A of Fig. 1G in the z-x cross-section. In some examples, the lateral offset of one strand segment 105A with respect to its adjacent strand segment 105A in the subsequent layer is equal to zero (offset= 0) along (or horizontal to) the x-y plane. In these cases, the baseline gap height, gh, may be simply equal to the average strand diameter, d or 2R.

Alternatively, as shown in Fig. 1I, there may be cases wherein the lateral offset of one strand segment 105A with respect to its adjacent strand segment 105A in the subsequent layer along or horizontal to the x-y plane is greater than zero. In such cases, the gap height, gh, may be expressed as

$$(11): gh = \sqrt{(offset)^2 + (2 \times lt)^2} - 2R$$

It may be referred to as a layer thickness. The dimension $2 \times lt$ may be the distance measured in the z-direction between a mid-point of the first strand segment 105A and the mid-point of the second adjacent strand segment 105A. The dimension $2 \times lt$, may be an offset in a z-direction, whereas the lateral offset may be an offset in a x or y direction. The lateral offset value between the adjacent strand segments may lie between 0 % and 99 % (or e.g. between 0 % and 50 %, or e.g. between 0 % and 10 %) of gap length, gl.

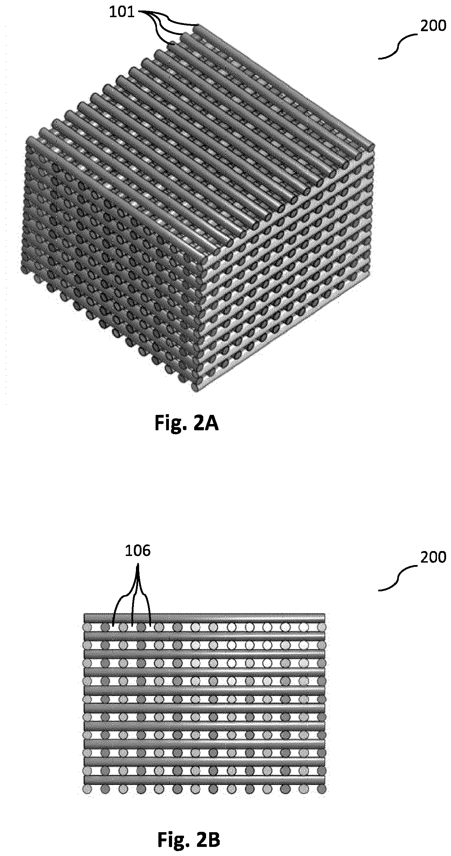

Figs. 2A and 2B show respectively perspective and cross-sectional side-view illustrations of an implant 200 for insertion into a patient. The implant 200 may include one or more or all of the features described in connection with Figs. 1A to 1E. The implant 200 may have different dimensions compared to the implant 100, such as a different gap height to gap length ratio. For example, the baseline gap height, gh, may be similar to the baseline gap length, gl (e.g. between 95% and 100% of the base line gap length).

The implant 100, 200 may be any type of implant suitable for insertion into a patient, which may be a living body (e.g. a human body, or e.g. an animal body). The implant 100, 200 may be a scaffold for bone tissue or implant for any soft-tissue part of the human or animal body. The implant might be a breast or thorax implant (the latter may be uses for treatment of pectus malformation such as pectus excavatum), or for other parts of the body, such as the gluteal region (also known as buttock), the calf, parts of the face such as a cheek, or a testicular implant. In this context, it is noted here, that pectus malformation such as pectus excavatum can affect both males and females and thus pectus excavatum of both a male and a female subject both can be treated by an implant of the invention. In line with the above, an implant of the invention can adopt any suitable form, merely depending on the tissue that is to be reconstructed or augmented. The implant may, for example, have the form of a gluteal implant as described in US patent 10,004,585. Dependent on the type of implant required, the geometry of the implant 100, 200 (e.g. the size and/or the shape of the implant) may be tailored to meet the criteria required of the implant.)

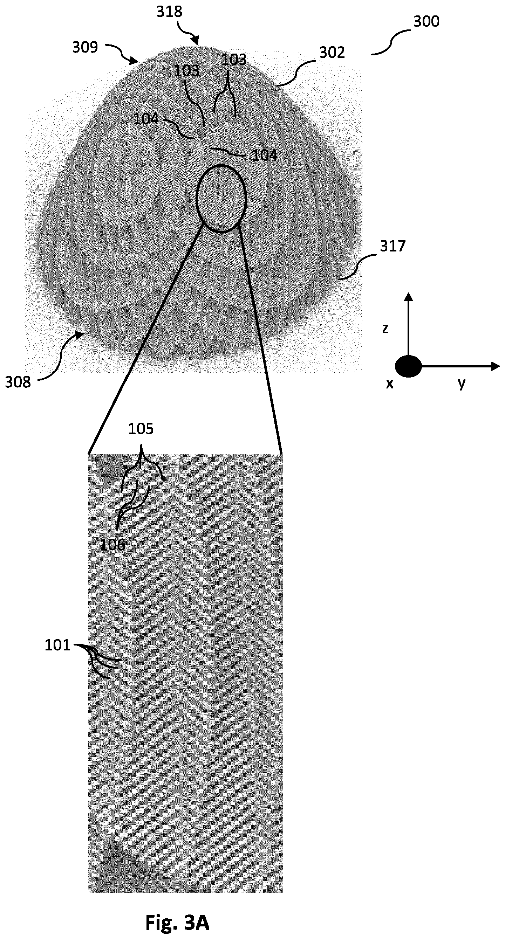

Fig. 3A shows a perspective illustration of an implant 300 suitable for use as breast implant. The implant 300 may include one or more or all of the features of the implants described in connection with Figs. 1A to 2B. Although the implant 300 is described with respect to breast implants, such an implant may also be valid for other parts of the body, such as the thorax, the gluteal region, the calf, or parts of the face such as a cheek (cf, above).

As shown in Fig. 3A, the implant 300 comprises a plurality of strands 101 forming a three-dimensional structure 302. The three-dimensional structure 302 comprises a plurality of hollow channels 103. Each hollow channel 103 comprises a plurality of sidewalls 104. A sidewall 104 comprises a plurality of strand segments 105 and a plurality of gaps 106 arranged alternatingly so that a gap 106 is formed between adjacent strand segments 105 of the sidewall 104. The plurality of gaps 106 are reversibly expandable gaps.

The three-dimensional structure 302 of the implant 300 may include a first outer surface region 308 and a second (different and/or opposite facing) outer surface region 309. It may be understood that an outer surface region may refer to (or may be) an outermost surface, an outermost layer, and/or an outermost contour of the implant 300. An outermost surface and outermost contour may be formed from one or more layers or lines. An outer surface region may refer to (or may be) an outermost group of layers (e.g. a single outermost layer, or e.g. an outermost plurality of layers) of the implant 300. An outer surface region may refer to an exterior facing surface of the implant 300.

The first outer surface region 308 of the implant 300 may include, or may have a first surface curvature. The first outer surface region 308 of the implant 300 may be the largest planar (or e.g. flattest) surface of the implant 300. For example, the first outer surface region 308 may be a flattest surface of the implant and/or surface with the least (or smallest) amount of curvature. As shown in Fig. 3A, the first outer surface region 308 of the implant 300 may be substantially parallel to a two-dimensional (x-y) cartesian plane. Alternatively, or optionally, a plane of best fit of the first outer surface region 308 may be parallel to the two-dimensional (x-y) cartesian plane.

The second outer surface region 309 may have a geometry (e.g. the shape, curvature, size) which represents a geometry of a patient's breast to be constructed by the implant 300. The second outer surface region 309 of the implant 300 may include, or may have a second surface curvature different to the first surface curvature. The second surface curvature may be greater than the first surface curvature. The second outer surface region 309 of the implant 300 may be contiguous to (e.g. abutting) the first outer surface region 308 of the implant 300 at a perimeter 317 (e.g. a circumference) of the first outer surface region 308. For example, second outer surface region 309 of the implant 300 may abut the first outer surface region 308, wherein the perimeter 317 of the first outer surface region 308 may be a shared edge (or interface) between the first outer surface region 308 and the second outer surface region 106. Additionally, or optionally, the second outer surface region 309 may include an apex region 318 at the second outer surface region 309.

The location (or position) of the apex region 318 at the second outer surface region 318 of the implant may be based on (and/or may coincide with) the location (or position) of the nipple/areola of the breast to be constructed by the implant 300.

Optionally, an acute tilt angle between one or more sidewalls and a reference axis (e.g. an x-axis) representing the first outer surface region may be less than 90 degrees (or e.g. less than 60 degrees). The reference axis may be based on a plane or line of best fit of the first outer surface region 108. Optionally or alternatively, the acute tilt angle between one or more sidewall 104s and the reference axis may be approximately 90 degrees (e.g. as shown in Figs. 1A to 2B, the channels may be perpendicular channels). The plurality of hollow channels 103 may consist of between 5 and 1000 hollow channels (or e.g. between 5 and 60 channels, or e.g. between 8 and 20 channels).

The three-dimensional structure 302 of the implant 300 may be a reversibly compressible three-dimensional structure 302. For example, the individual unit cells of the implant 300 may be spring-like unit cells. A spring-like unit cell may be compressible to at least 80 % (or e.g. at least 70 %, or e.g. at least 60 %, or e.g. at least 50 %, or e.g. at least 30 %) of its original volume. By being reversibly compressible, each unit cell may be able to recover or return to its original (resting) volume after a compression force has been removed (even at the same ambient pressure and temperature). A reversibly compressible spring-like unit cell may be configured to recover to between 80 % and 100 % (or e.g. between 95 % and 100 %, or e.g. between 98 % and 100 %) of its original volume, after the compression force exerted on the implant 300 is removed.

The softness of the implant 300 may be represented by a c-value. The c-value may be expressed by the formula

$$c = \frac{F_{20\,\%} - F_{10\,\%}}{\varepsilon_{20\,\%} - \varepsilon_{10\,\%}}$$

$F_{20\,\%}$ is the force value, in N, at a compression of 20 %, $F_{10\,\%}$ is the force value, in N, at a compression of 10 %, $\varepsilon_{10\,\%}$ is the strain value at a compression of 10 %, and $\varepsilon_{20\,\%}$ is the strain value at a compression of 20 %. A c-value representing a softness of the implant 300 may lie between 20 N and 190 N (or e.g. between 20 N and 150 N, or e.g. between 30 N and 100 N, or e.g. between 30 N and 40 N), for example.

A material density, $\rho$, of the implant 300 may lie between 0.1 gr/cm$^3$ and 2 gr/cm$^3$. (or e.g. between 0.1 gr/cm$^3$ and 1 gr/cm$^3$, or e.g. between 0.1 gr/cm$^3$ and 0.5 gr/cm$^3$). The material density may be determined by the weight of the implant 300 divided by the resting volume of the implant before insertion into the patient. In comparison, the material density of silicone is 0.98 gr/cm$^3$, and the material density of saline is 1.005 gr/cm$^3$. Thus, the weight of the implant of 300 may be between 10 % and 20 % (or e.g. between 10 % and 15 %) of its volume value in milliliters, and between 10 % and 20 % (or e.g. between 10 % and 15 %) of traditional non-porous silicone/saline implants (whose weights in grams are roughly the same as their volume value in milliliters). As an example, an implant 300 having a volume of 250ml may weigh 25g, whereas a traditional silicone implant having a volume of 250ml, may weight 240g, and a saline implant having a volume of 250 ml, may weigh 250g. One or more or all of these features leads to an implant having a lightweight scaffold, wherein a weight reduction of 90 % compared to traditional implants may be achieved.

The plurality of strands 101 of the implant 300 may be formed from a polymer material such as a surface-degradable polymer. A surface-degradable polymer material may include or may be a polymer material that degrades predominantly via the surface degradation mechanism as opposed to bulk degradation. The plurality of strands 101 may include or may be formed from or made of biodegradable material. The biodegradable material may be selected from the group consisting of polycaprolactone, poly(1,3-trimethylene carbonate), polylactide, polyglycolide, poly(ester amide), poly(ethylene glycol)/poly(butylene terephthalate), poly(glycerol sebacate), poly(1,8-octanediol-co-citric acid), poly(1,10-decanediol-co-D,L-lactic acid), poly(diol citrate), poly(glycolide-co-caprolactone), poly(1,3-trimethylene carbonate-co-lactide), poly(1,3- trimethylene carbonate-co-caprolactone) and a copolymer of at least two of these materials. Optionally, the biodegradable material may be polycaprolactone. Optionally, the biodegradable material may be a copolymer of polycaprolactone and either poly-trimethylene carbonate or polylactide. Alternatively, the plurality of strands 101 may include a non-degradable material such as nylon. The thickness (or diameter) of the plurality of strands 101 may be selected so that the strand is flexible. Bulk PCL, for example, may have an elastic modulus (E) of 216 MPa, a tensile strength of 10 MPa, and a breaking stress of 26.5 MPA.

The equation (10): $\frac{R}{g_l^2} \leq \frac{\sigma_{yield}}{12E\delta}$ may be applied in the design of the implants described herein. $\frac{R}{g_l^2}$ may be selected based on the material properties of a material (such as Young's modulus and yield strength) for forming the implant, and desired deflection capacity $\delta$ based on surgical requirements, such as the size of a needle used by a surgeon.

For example, a material used for forming the implant 300 may have an Elastic modulus of 270 MPa, and a yield strength $\sigma_{yield}$ of 12.5 MPa. A needle to be used may have a diameter of 2 mm.

Geometrical properties related to the implant, such as lateral offset, layer thickness, radius and gap length may be chosen. For example, lateral offset may be 1 mm, a layer thickness, lt, may be 0.2 mm, a radius, R, may be 0.175 mm and a gap length, gl may be 6 mm.

Using equation (11): $gh = \sqrt{(offset)^2 + (2 \times lt)^2} - 2R$ , a value for gap height may be calculated or determined, wherein $gh = \sqrt{(1)^2 + (2 \times 0.175)^2} - 2 \times 0.175 = 0.709$ .

Using equation (8): $\delta = \frac{nd - gh}{2}$ , a value for deflection capacity may be calculated or determined, wherein

$$\delta = \frac{2 - gh}{2} = \frac{2 - 0.709}{2} = 0.6452$$

Using equation (10), a maximum limit for $\frac{R}{gl^2}$ may be calculated, wherein $\frac{\sigma_{yield}}{12E\delta} = \frac{12.5}{12 \times 270 \times 0.6452} = 0.005979$.

$\frac{R}{gl^2} = \frac{0.175}{6^2} = 0.004861$ , which is less than the maximum limit $\frac{\sigma_{yield}}{12E\delta} = 0.005979$ .

Additionally, or optionally, the implant 300 may further include a plurality of contouring strands 319 arranged at an outer surface region (e.g. at the second outer surface region 309) of the implant 300.

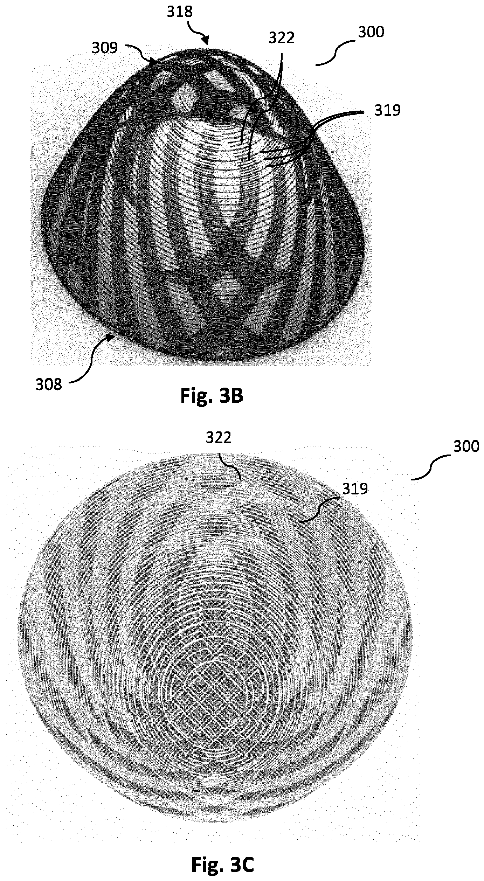

Figs. 3B and 3C shows a perspective side view illustration and a perspective top view illustration of an implant 300 further including a plurality contouring strands 319 and surface filler strands 322.

As shown in Figs. 3B and 3C, the plurality of strands forming the implant 300 may include a plurality of contouring strands 319 and and surface filler strands 322. Each contouring strand 319 may form a semi-contour around the second outer surface region 319. The semi-contour of the contouring strands 319 may extend only partially (e.g. between 30 % and 80 %, or e.g. between 40 % and 70 %, or e.g. between 50 % and 70 %) around the perimeter of a layer. The plurality of contouring strands 319 may be arranged consecutively (e.g. successively) between the first outer surface region 308 and the apex region 318. The plurality of contouring strands 319 may further be arranged such that adjacent contouring strands 319 are separated by a reversibly expandable gap 106. For example, a reversibly expandable contouring gap 106 may be formed between adjacent strand segments of the plurality of contouring strands 319.

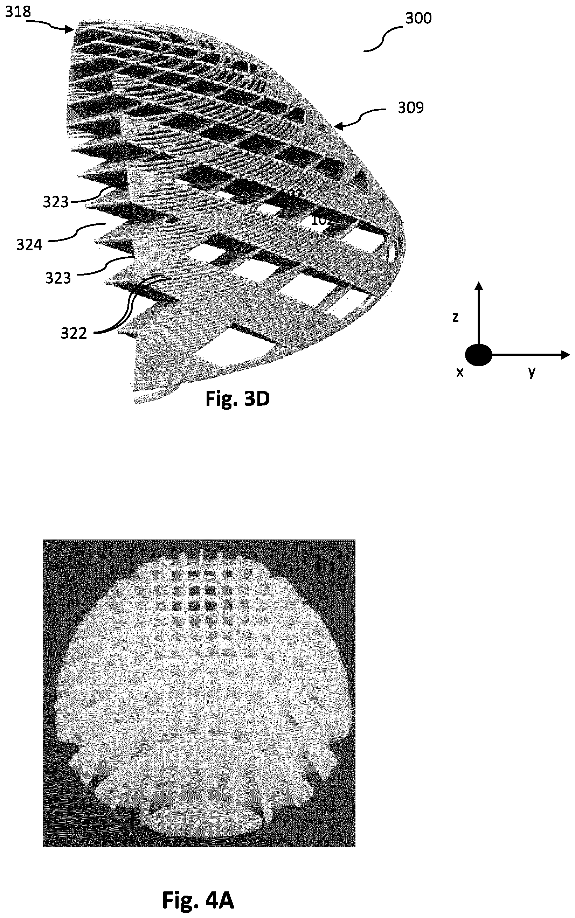

As shown in Fig. 3D, the implant 300 may further include surface filler strands 322 formed at the outermost surface of the three-dimensional structure 302. Optionally, the plurality of surface filler strands 322 may be arranged in columns 323 (or strips) at the second outer surface region. Each surface filler column 323 may include a plurality of surface filler strands 322 and a plurality of reversibly expandable surface gaps. Each reversibly expandable surface gap may be formed between adjacent surface filler strands 322 of a column 323. A surface filler column 323 may be arranged adjacent to an open column 324. An open column may include (or may be) a column of surface at the that are free from surface filler portions. Optionally, a plurality of surface filler columns 323 and a plurality of open columns 324 may be arranged alternatingly at the outer surface regions (e.g. at the second outer surface region 309). Optionally a plurality of surface filler columns 323 and a plurality of open columns 324 may be arranged in a criss-cross fashion at the outer surface region.

The plurality of hollow channels 103 may extend through the bulk of the implant 300. Adjacent strand segments may be separated by reversibly expandable gaps.

The implant 300 may be inserted into the breast region of a patient. After the insertion of the implant 300 into the patient, fat injection may need to be performed. Fat injection may be necessary because the regenerated tissue which "regrows" inside the implant has been observed to be stiffer than the natural breast tissue. Therefore, in order to make the final outcome as soft as natural breast tissue, a suitable percentage of fat may be collected, e.g. by means of liposuction, and injected into the implant 300 using a specific cannula (or needle). Due to reversibly expandable gaps 106 of the implant 100, problematic aspects of the injection procedure (e.g. injections which perforate the structure several times and damage the strands and affect the integrity of the overall structure of the implant 300) may be avoided.

The implant 300 may be an implant for use in guiding a needle through the implant for implant surgery. The implant 300 may be configured to receive an elongated object (such as a needle for fat injection) into the bulk of the three-dimensional structure 102. The elongated object may have a diameter larger than a baseline gap height, gh, of the gaps 106 of the implant. For example, the diameter of the elongated object may be between 110% and 250% times the baseline gap height, gh, of the gaps 106 of the implant. Additionally, the elongated object may have a length greater than at least two times (or five times, or 10 times) a width of a hollow channel. The plurality of strands may be configured so that a height of the gap 106 increases due to the object being received by the gap, and the height of the gap 106 decreases due to the object being removed from the gap 106. Such a needle or cannula may have a diameter of at least 0.8 mm (or e.g. at least 2mm, or e.g. at least 4 mm, or e.g. between 0.8mm and 4mm, or e.g. between 0.8mm and 3mm), and a length of at least 2 cm. More than 80 % (or e.g. more than 90 %, or e.g. all) of all the gaps of the implant 300 having a baseline gap height of less than 1 mm may be reversibly expandable gaps.

Fig. 4A shows an implant without the reversibly expandable gaps of implants 100, 200, 300.

Unlike the implants 100, 200, 300 with reversibly expandable gaps 106, the implant of Fig. 4A would require that the surgeon insert the fat injection needle at precise locations. For example, after inserting the implant into the patient, the surgeon would have to visually locate the largest openings of the implant. These largest openings may be the channel pores defining the channel ends, whose pore size area may be at least 2 times larger than the gap area. Then, the surgeon would have to insert the needle precisely into the pores and along the channel length (e.g. along the longitudinal axis of the channel). If the surgeon is unable to visually locate the channel ends, or inserts the needle into the implant at an angle that is not along the channel length, or inserts the needle into a sidewall of the channel, the injections from the needle may damage the strands of the implant and/or damage the three-dimensional structure of the implant.

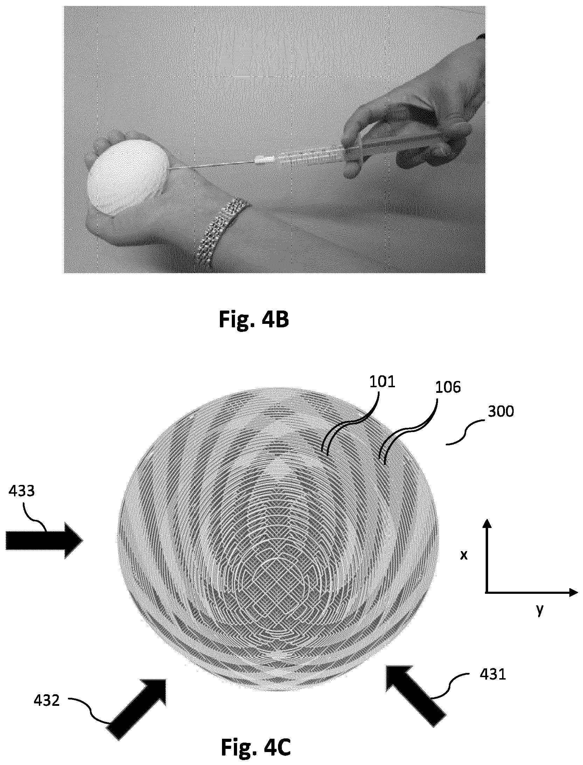

Fig. 4B shows an image of a cannula for fat injection being inserted into the implant 300. With the implant 300, it is possible for the surgeon to blindly insert the needle into the implant 300 from anywhere (e.g. any surface) of the implant. Due to the implant 300 having the reversibly expandable gaps 106, the needle may be inserted into the breast implant from any direction at the second outer surface region 309 of the implant, and/or the needle may be inserted through any sidewall 104 within the bulk of the implant 300. The needle may be first inserted through the bulk internal structure of the implant 300 from an insertion region of the implant 300. The insertion region may be a randomly selected region of the implant. The needle may be inserted such that the needle concurrently crosses and/or enters several (e.g. a plurality of) hollow channels 103, and penetrating through several (e.g. a plurality of) sidewalls. In other words, the needle may be concurrently inserted through a plurality of hollow channels 103. The needle may then be withdrawn from the implant 300 in a step-wise function over a plurality of withdrawal steps. After each withdrawal step, fat from the needle may be injected into the implant. The alternating processes of withdrawing the needle and fat-injection, may be repeated until the needle is fully withdrawn from the implant. This process starting with blind injection may be carried out repeatedly from a plurality of random insertion regions. The number of times (e.g. at least 20 times, or e.g. at least 30 times, or e.g. at least 60 times) the process is carried out may be based on the number of injections required and/or the amount of fat injection required. The needle may need to be inserted very often during surgery. The implant 300 allows the surgical process to be sped up, since the surgeon no longer needs to visually locate each channel and opening before inserting the needle, and can instead carry out blind insertions. Furthermore, the implant 300 allows the cannula to penetrate a plurality of sidewalls 104 and channels 103 concurrently. This also allows the surgical process to be sped up. Furthermore, since the gaps 106 are able to accommodate the entry of the cannula, neither the implant 300 not the cannula suffers from damage. Thus, the inserted implant 300 has an improved structural integrity compared to implants 300 which have not implemented reversibly expandable gaps.

Fig. 4C shows an example of possible regions of insertions for multi-injections testing. A certain number of injections (e.g. 60 injections) may be divided over 3 broad injection sites (431, 432, 433). For example, 20 injections may be performed over each region. The injections (e.g. the insertion of the needle) may be carried out so that the needle may be inserted at any angle relative to the first outer surface region 308 of the implant. For example, the needle may be inserted in a flat plane, or at an angle of inclination or declination with respect to the first outer surface region 308.

The plurality of strands 101 may be configured such that the gaps 106 of the implant 300 are reversibly expandable when the implant is at rest and even when the implant 300 is under compression (for example, even when the implant 300 is inside the body. An implant under compression may be an implant which has been compressed to less than 90 % (or e.g. less than 70 %, or e.g. less than 60 %, or e.g. less than 50 %, or e.g. less than 30 %) of its resting volume. A height of the gap 106 may increase to greater than 110 % (or e.g. greater than 150 %, or e.g. greater than 200 %, or e.g. between 120 % and 250 %, or e.g. between 120 % and 200 %) of the baseline gap height, gh, and/or the average strand diameter, d, due to the object being received by the gap 106. The reversibly expandable gap 106 may be configured to recover or return (or decrease) to at least 110 % (or e.g. to at least 105 %, or e.g. to 100 %, or e.g. between 80 % and 115 %, or e.g. between 90 % and 110 %) of its original gap height, gh, and/or average strand diameter, d, due to the object being removed from the gap 106.

To accommodate each insertion of the needle, the plurality of strands 101 may be arranged such that the reversibly expandable gaps 106 of at least two (or e.g. more than two, or e.g. more than three, or e.g. more than five) consecutive hollow channels may be concurrently expandable due to the object being received by the gaps of the at least two consecutive hollow channels concurrently. The plurality of strands 101 may be arranged such that reversibly expandable gaps 106 of at least two (or e.g. more than two, or e.g. more than three, or e.g. more than five) different sidewalls 104 of the implant 300 may be concurrently expandable due to the object being received by the gaps of the at least two (or e.g. more than two, or e.g. more than three, or e.g. more than five) different sidewalls concurrently. The expansion of the gaps 106 may occur even without increasing or changing the overall resting volume (or construction volume) of the implant 300. In other words, the reversibly expandable gaps may be configured to expand, while the overall volume of the implant 300 remains constant or even under compression.

The surgeon inserting the needle should be able to penetrate the bulk of the implant without damaging the structure. For example, the filaments enclosing the gap may bend, stretch, or move out of the way and not break. Furthermore, the surgeon may exert not more than 50 N (or e.g. between 2 N and 50 N, or e.g. between 5 N and 20 N, or e.g. between 5 N and 10 N) of force to insert the needle through a gap. For example, upon being subjected to 10 N from the injection needle, the filaments reversibly deform (elongate or stretch or move) to allow the needle to pass through the gap. The material itself may be selected so that the filaments do not break at this elongation point.

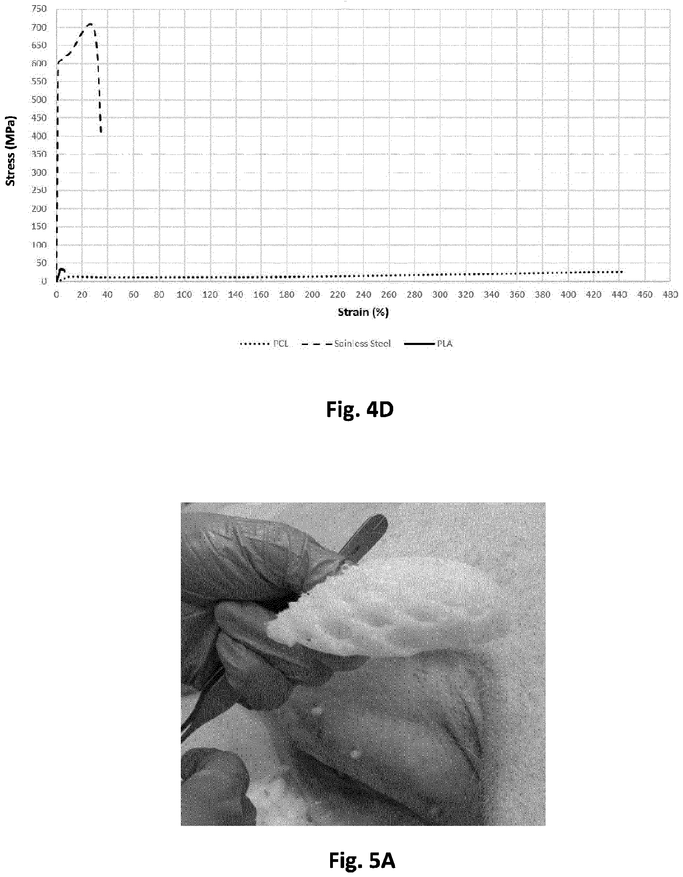

Fig. 4D shows a stress strain curves of strands comprising different materials, such as stainless steel, polylactic acid plastic PLA and Polycaprolactone PCL.

A fracture point of a stainless steel strand may be at a strain of 32% under a stress of 720 MPa. A fracture point of a PLA strand may be at a strain of 5% when subjected to a stress of 40 MPa. The fracture point strain value for PCL strands is 460% (under a stress of less than 50 MPa), which is approximately 15 times more than stainless steel strands and around 70 times more than the PLA strands. PCL accept high amount of deformation because of high Rupture strain, it means that it can be extended at least 4 times of its initial length.

The material for forming the plurality of strands may be selected such that it has a fracture point on a stress-strain diagram, wherein a strain at the fracture point is larger than 30% and stress at the fracture point is less than 250 MPa.

The material of the strands of the implant may exhibit similar stress-strain behaviour as PCL. For example, the fracture point on the stress-strain diagram may be above 30 % (or e.g. above 100 %, or e.g. above 200 %, or e.g. above 300 %, or e.g. above 400 %), and the stress may be below 50 MPa, or between 10 MPa and 250 MPa (or e.g. between 10 MPa and 100 MPa, or e.g. between 10 MPa and 50 MPa, or e.g. between 10 and 30 MPa). Optionally, a fracture point of a strand of the plurality of strands may be larger than 30 % (or e.g. above 100 %, or e.g. above 200 %, or e.g. above 300 %, or e.g. above 400 %) and the corresponding stress may be below 50 MPa (or e.g. between 10 MPa and 250 MPa, or e.g. between 10 MPa and 100 MPa, or e.g. between 10 MPa and 50 MPa, or e.g. between 10 and 30 MPa).

As an example, if the distance between two neighboring strands varies between 0.56 mm and 1.84 mm and the needle diameter is 2 mm, during the penetration of the needle to the walls, PCL may deform easily without reaching to its rupture point. To the contrary, PLA and Stainless steel cannot take such a deformation and they would reach to the rupture point while needle penetration.

Fig. 5A shows an implant, such as the implant 300 after a mock surgery, During the mock surgery, the implant 300 was inserted inside a pig. The implant 300 was injected blindly (from random positions) with fat. Upon removal, it was shown that the three-dimensional scaffolding structure 302 of the implant 300 was filled with fat, and that the fat was distributed homogeneously despite the blind (random) injections.

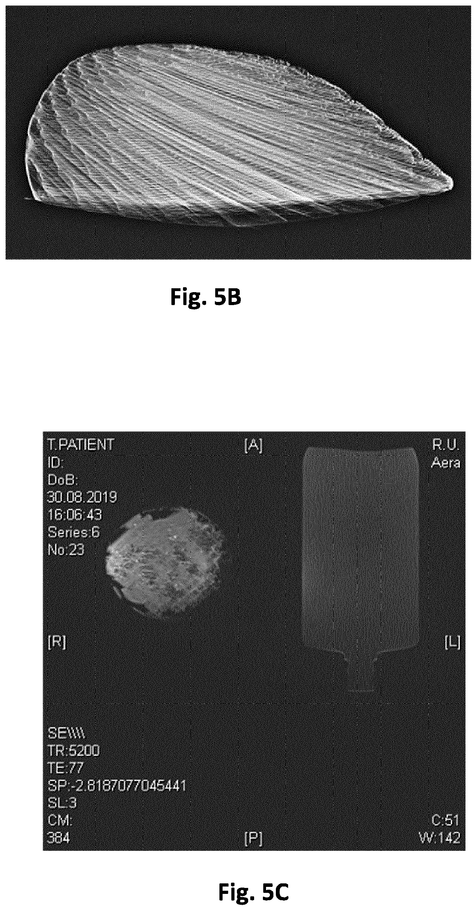

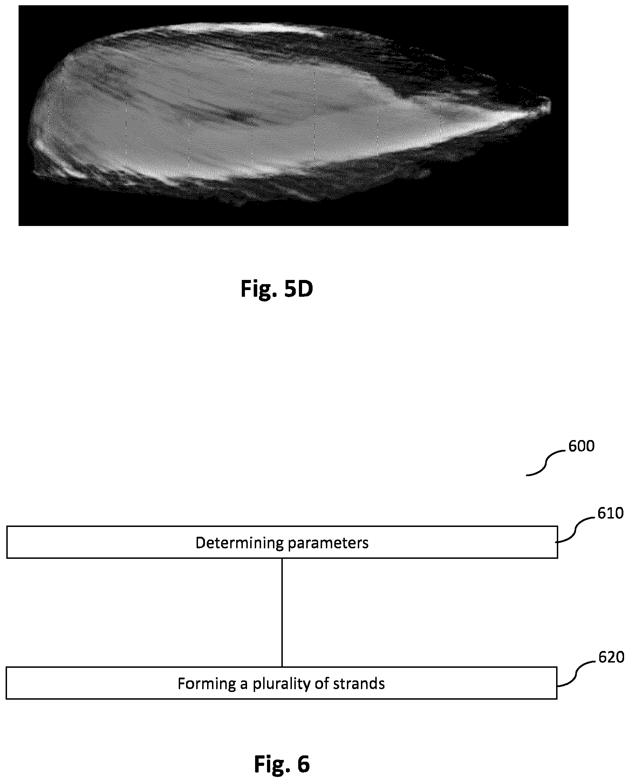

Figs. 5B, 5C and 5D show respectively images of the implant 300 after the blind injections. Fig. 5C shows that fat is equally distributed in the implant 300. The white colour represents fat and the black colour represents air. Mechanical testing (e.g. tensile, compression and shear testing) was carried out to evaluate the change in dimensions and/or mechanical properties of the scaffold before being subjected to fat injection. After tensile testing (by rubbing) for 11112 cycles at a frequency of 0.4 Hz, the dimensions (width, projection and height) and the softness (c-value) and the strength of the scaffold in shear (Fmax) were still within the specifications of the implant. In other words, the tensile strength of the implant 300 was not damaged. Comparing the softness indicator (which is a good representation of the integrity of the scaffold) after the test to results before the test, a reduction of only roughly 10 % was detected. Thus, although the scaffolds had a reasonable and expected loss in mechanical properties, the integrity of the scaffold remained unaffected.

Fig. 6 shows a flow chart of a method 600 for forming an implant that was described previously..

The method 600 comprises forming 620 a plurality of strands to form a three-dimensional structure, The plurality of strands are formed from a material having an a yield strength ($\sigma_{yield}$) and elastic modulus (E). The three-dimensional structure comprises a plurality of hollow channels. Each hollow channel comprises a plurality of sidewalls. A sidewall comprises a plurality of gaps and a plurality of consecutive strand segments of the plurality of strands. The plurality of strand segments and the plurality of gaps are arranged alternatingly so that a gap is formed between adjacent strand segments of the sidewall, wherein the gap comprises a gap length (gl) and a resting gap height (gh). The plurality of gaps are reversibly expandable gaps. The adjacent strand segments of a reversibly expandable gap comprise a deflection capability ($\delta$) in response to an object being received by the gap so that an increased gap height is attained between the adjacent strand segments. The gap returns towards the resting gap height in response to the object being removed from the gap. A radius (R) of the plurality of strands and respective gap lengths (gl) of the plurality of gaps are based on the yield strength and the elastic modulus of the material.

Forming 620 the plurality of strands may include sequentially printing a plurality of layers, wherein a layer comprises a lattice arrangement of two-dimensional unit cells. The plurality of layers may be arranged such that aligned unit cells of consecutive layers of the plurality of layers form a hollow channel of the plurality of channels. The implant may be formed by sequentially forming (or e.g. 3-D printing by fused deposition modelling) layers to form the three-dimensionally (3D) printed scaffold structure. The printing may be carried out, layer by layer in a print direction (e.g. in the z-direction), so that a sequential arrangement of successive layers are formed in the print direction. The sequential arrangement (by printing) of layers on top of each other may lead to the forming of the three-dimensional structure, with the edges (or perimeter) of the plurality of layers defining the shape and/or geometry of the implant to be formed. Alternatively, the plurality of strands may be formed by any three-dimensional printing method, such as selective laser sintering (SLS). Optionally, the three-dimensional structure may be formed by a printing process based on more than three dimensions of movement, e.g. a five-dimensional (5D) printing process, or a six-dimensional (6D) printing process.

The method 600 may optionally further include determining 610 at least one of the following parameters (e.g. before forming 620 the plurality of strands). The parameters to be determined may be a number of hollow channels of the three-dimensional structure to be formed, a number of layers of the three-dimensional structure to be formed, and dimensions of unit cells to be formed. Additionally, the parameters to be determined may be a gap length, gl, between adjacent strands within a layer, and a gap height, gh, of the gaps between adjacent strands within a sidewall of a channel. Additionally, the parameters to be determined may include a diameter, d, of the plurality of strands to be formed.

Determining 610 the parameters may include determining a deflection capability ($\delta$) of a reversibly expandable gap based on an object to be received by the reversibly expandable gap. Determining 610 the parameters may further include determining material properties of the plurality of strands to be formed, wherein the material properties comprises the yield strength ($\sigma_{yield}$) and a young's modulus (E) of the material. Determining 610 the parameters may further include determining a radius (R) and gap length (gl) for respective strand segments of the plurality of strand segments to be formed. Determining 610 the parameters may further include determining a number of hollow channels of the three-dimensional structure to be formed, a number of layers of the three-dimensional structure to be formed and/or dimensions of the unit cells.

The plurality of strands may be formed after determining the parameters, such that a three-dimensional structure comprising the reversibly expandable gaps is formed. For example, the parameters may be determined, such that

$$\frac{R}{g_l{}^2} \leq \frac{\sigma_{yield}}{12E\delta}$$

. The three-dimensional structure formed may include at least one of the following parameters: the determined number of hollow channels, the determined number of layers, the determined dimensions of the unit cells, the determined gap length, gl, between adjacent strands within a layer, the determined gap height, gh, of the gaps between adjacent strands within a sidewall of a channel, the determined diameter of the plurality of strands. The three-dimensional structure formed by the method 600 may be three-dimensional structure of any of implants 100, 200, 300 described in connection with Figs. 1A to 5C.

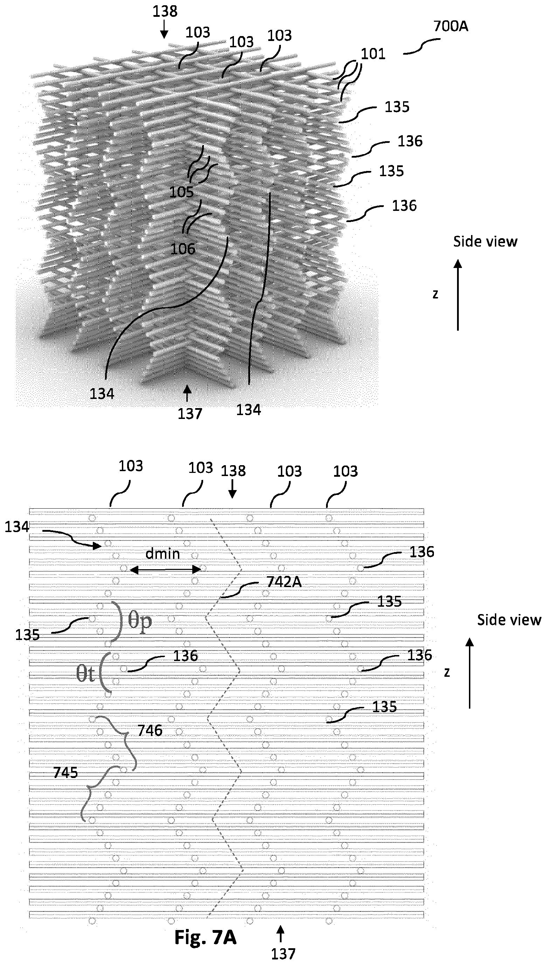

Figs. 7A to 7G shows examples of implants 700A to 700F including channels with oscillating (e.g. undulating, or e.g. zig-zag, or sinusoidal) sidewalls. The implants 700A to 700F may include one or more or all of the features already described in connection with Figs. 1A to 6. For example, the implants 700A to 700F may include the reversibly expandable gaps described in connection with the implants of Figs. 1A to 6.

Fig. 7A shows a three-dimensional soft-tissue implant 700A for insertion into a patient. The implant 700A comprises a plurality of strands 101 forming a three-dimensional structure. The three-dimensional structure comprises a plurality of hollow channels 103. Each hollow channel 103 comprises a plurality of intersecting sidewalls 134. Each sidewall 134 comprises a plurality of strand segments 105 and a plurality of gaps 106 arranged alternatingly. At least one sidewall 134 of the hollow channel 103 is an undulating (e.g. oscillating, zig-zag and/or sinusoidal sidewall). Optionally, each hollow channel 103 may include a first undulating sidewall 134 and a second undulating sidewall 134 facing opposite to the first undulating sidewall 134.

A hollow channel 103 with at least one undulating sidewall may be referred to as an undulating (e.g. zig-zag and/or sinusoidal) channel. A (or each) zig-zag (or sinusoidal) channel 103 may be a channel in which at least two opposite sidewalls of a channel are arranged with respect to each other so that the channel zig-zags between a first end 137 (proximal end) of the channel towards (or to) a second end 138 (distal end) of the channel 103. For example, a zig-zag channel 103 may have a first zig-zag sidewall 134 and a second zig-zag sidewall 134 opposite the first zig-zag sidewall. Each zig-zag sidewall 134 of a zig-zag channel may comprise a plurality of strands 101 arranged sequentially in the z-direction. The plurality of strands 101 may be substantially parallel to each other, and perpendicular to the z-direction. The sequentially arranged strands 101 of the zig-zag sidewall 134 may be arranged to have a lateral offset with respect to each other, and so as to create the pattern of peaks 135 and troughs 136 along the sidewall 134. The plurality of strand segments of the undulating sidewall belong to different layers of the implant. Adjacent strand segments of the undulating sidewall are separated by a gap (e.g. a reversibly expandable gap described in any of Figs. 1 to 6), and have a lateral offset with respect to each other to create a pattern of a plurality of peaks and a plurality of troughs of the undulating sidewall.

Each hollow channel is formed by at least three sidewalls contiguous with each other. The peaks and troughs of the undulating sidewall are formed by the lateral offset between adjacent strand segments of the plurality of strand segments of the sidewalls. The peaks and troughs are arranged alternatingly between a first distal end of the channel and a second distal end of the channel.

An undulating portion (e.g. a zig-zag or sinusoidal portion) may be understood to be a portion that has a plurality of peaks (maximums) 135 and troughs (minimums) 136 arranged alternatingly from the first end 137 of the channel 103 to the second end 138 of the channel 103 (and/or between the first end 137 of the channel 103 to the second end 138 of the channel 103). These peaks 135 and troughs 136 may be viewed from a vertical cross-section through the sidewall (e.g. through the z-direction). Such a vertical cross-section may be perpendicular to the strand direction and may be a cross-section through and/or parallel to the channel length.

A peak 135 (and/or trough 136), may be formed from the strands of the zig-zag sidewall 134 being arranged such that an angle θ (e.g. θt or θp) forms between a first plurality of consecutive strands 745 and a directly adjacent second plurality of consecutive strands 746 of the zig-zag sidewall 134. The angle θt may be the angle formed between a first plurality of consecutive strands 745 and a directly adjacent second plurality of consecutive strands 746 at the trough of the undulating portion, and the angle θp may be the angle formed between a second plurality of consecutive strands 745 and a directly adjacent (third) second plurality of consecutive strands at the peak of the undulating portion. An undulation portion may be a portion wherein θt and/or θp is less than 180 °. For contrast, a straight sidewall may be one in which θ is 180 ° along the entire channel length.

An undulating portion of the channel 103 may include at least one θt and/or θp angle that is less than 180 °. Optionally, the undulating portion of the zig-zag channel 103 may include between 1 and 1000, (or e.g. between 2 and 50, or e.g. between 2 and 25) peaks and troughs.

Fig. 7A shows an example of an implant 700A. Fig. 7A includes a three-dimensional perspective cross-section (top image) and a two-dimensional cross-section (bottom image) of the implant 700A. The implant 700A includes at least one undulating sidewall portion, wherein the undulating portion is a zig-zag portion.

In the example of Fig. 7A, the geometry of the first zig-zag sidewall portion 134 and the second zig-zag sidewall portion 134 may be dependent (or e.g. identical, or e.g. similar to each other) on each other, ignoring manufacturing differences. The use of the term "similar" may be understood to mean that the first zig-zag sidewall portion 134 and the second zig-zag sidewall portion 134 (and/or the zig-zag longitudinal axis 742A) may have one or more or all of the same features as each other, and/or that the first zig-zag sidewall and the second zig-zag sidewall may have identical or similar geometries or features over more than 80 % (or e.g. more than 90 %, or e.g. more than 95 %, or e.g. 100 %) of a channel length at least one of the first zig-zag sidewall 134 and the second zig-zag sidewall 134. One such feature may be the number of peaks 135 and troughs 136 along the channel length. Another such feature may be the peak-to-trough height, which may be a vertical height between a peak and a directly adjacent trough. Another such feature may be the peak-to-peak width between directly consecutive peaks. Another such feature may be the trough-to-trough width between directly consecutive troughs.

For example, the first zig-zag sidewall 134 and the opposite second zig-zag sidewall 134 may have similar pattern of peaks 135 and troughs 136. For example, the arrangement of θt and θp of the first sidewall portion 134 may be identical to the arrangement of θt and θp of the second sidewall portion 134. Additionally or optionally, the peaks 135 and troughs 136 of the first zig-zag sidewall portion 134 and of the second zig-zag sidewall portion 134 may be arranged with respect to each other so that a (minimal or smallest) diameter between the first zig-zag sidewall portion 134 and the second zig-zag sidewall portion 134 along the length of the channel is constant. For example, the (minimal or smallest) diameters between the first zig-zag sidewall and the second zig-zag sidewall along the channel length may have a deviation of less than 10 %. Furthermore, the zig-zag longitudinal axis 742A may have a similar (or same) geometry as at least one of the first zig-zag sidewall 134 and the second zig-zag sidewall 134 (e.g. both sidewalls).

The first zig-zag sidewall portion 134 and the second zig-zag sidewall portion 134 may be similar (or identical) along at least 80 % (or e.g. at least 90 %, or e.g. at least 95 %, or e.g. 100 %) of a channel length at least one of the first zig-zag sidewall portion 134 and the second zigzag sidewall portion 134. For example, the relative lateral offsets (x-direction) between consecutive strands of the first zig-zag sidewall 134 may be the same as the relative lateral offsets (x-direction) between consecutive strands of the second zig-zag sidewall 134, ignoring differences due to manufacturing along the at least 80 % of the channel length of at least one of the first zig-zag sidewall portion and the second zig-zag sidewall portion.

A longitudinal axis may include, or may be defined by a plurality of mid-points along the channel length of a channel 103. The plurality of mid-points may be the middle points of the (minimal or smallest) diameter, dmin, between the first sidewall and the second sidewall along the channel length. The peaks 135 and troughs 136 of the first zig-zag sidewall and of the second zig-zag sidewall may be arranged with respect to each other so that the zig-zag longitudinal axis 742A extends between the first end of the channel and the second end of the channel. The zig-zag longitudinal line 742A may have a similar pattern as the first zig-zag sidewall and the second zig-zag sidewall.

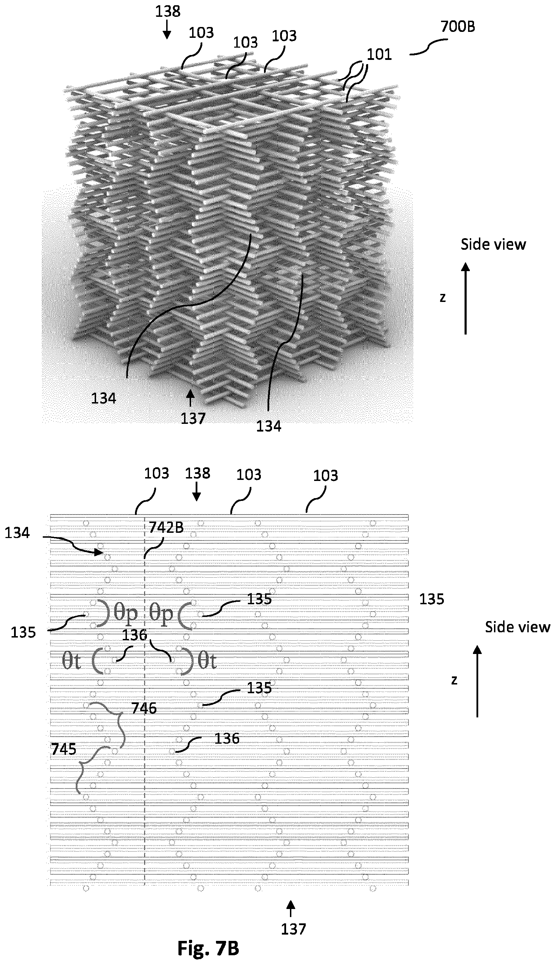

Fig. 7B shows an example of a further implant 700B. Fig. 7B includes a three-dimensional perspective cross-section (top image) and a two-dimensional cross-section (bottom image) of the implant 700B. Implant 700B may be similar to implant 700A and include one or more or all of the features of implant 700A. However, in the case of implant 700B, the second sidewall portion 134 may be a mirror-image of the first second sidewall portion 134 about the longitudinal axis 742B.

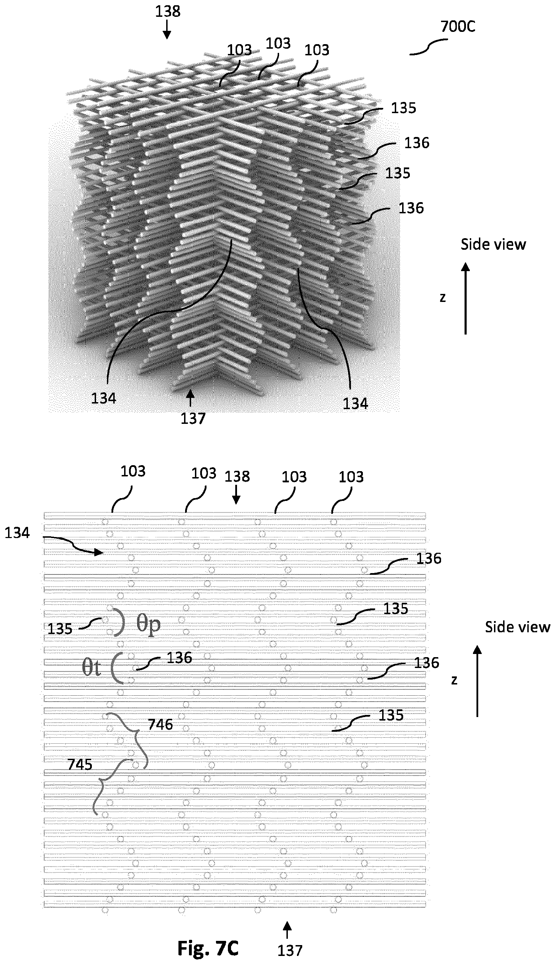

Fig. 7C shows an example of a further implant 700C. Fig. 7C includes a three-dimensional perspective cross-section (top image) and a two-dimensional cross-section (bottom image) of the implant 700C. Implant 700C may be similar to implant 700A and include one or more or all of the features of implant 700A. However, in the case of implant 700C, the undulating portions of the channel 103 are sinusoidal instead of zig-zag.

In a sinusoidal sidewall portion, a tangential line along the first plurality of consecutive strands 745 may be a varying tangential line (e.g. changing gradually, e.g. decreasing gradually) and a tangential line along the second plurality of consecutive strands 746 may also be varying tangential line (e.g. changing gradually, e.g. increasing gradually). In a zig-zag sidewall portion, a tangential line along the first plurality of consecutive strands 745 may be a constant tangential line (e.g. fixed tangential value, e.g. positive tangent) and a tangential line along the second plurality of consecutive strands 746 may also be constant tangential line (e.g. fixed tangential value, e.g. negative tangent).

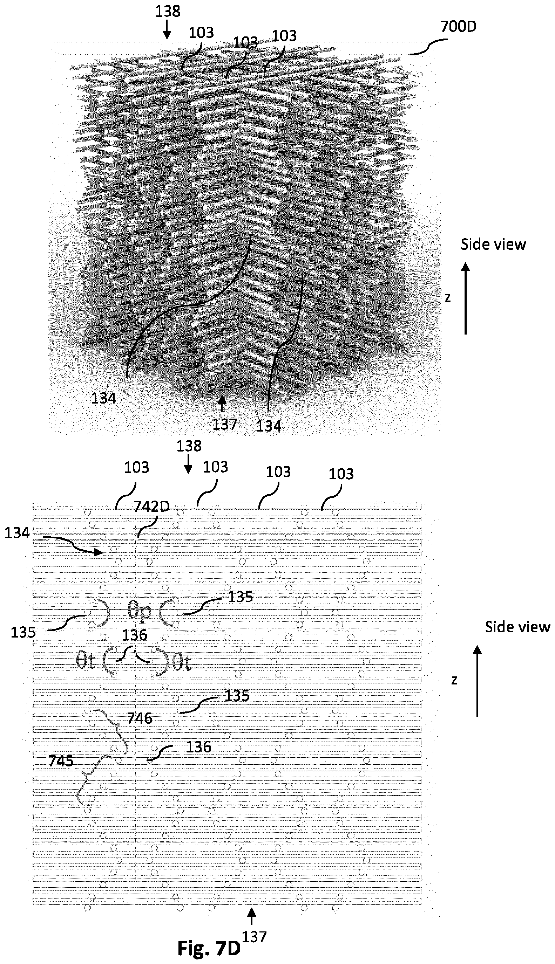

Fig. 7D shows an example of a further implant 700D. Fig. 7D includes a three-dimensional perspective cross-section (top image) and a two-dimensional cross-section (bottom image) of the implant 700D. Implant 700D may be similar to implant 700C and include one or more or all of the features of implant 700C. However, in the case of implant 700D, the second sidewall portion 134 may be a mirror-image of the first second sidewall portion 134 about the longitudinal axis 742D.

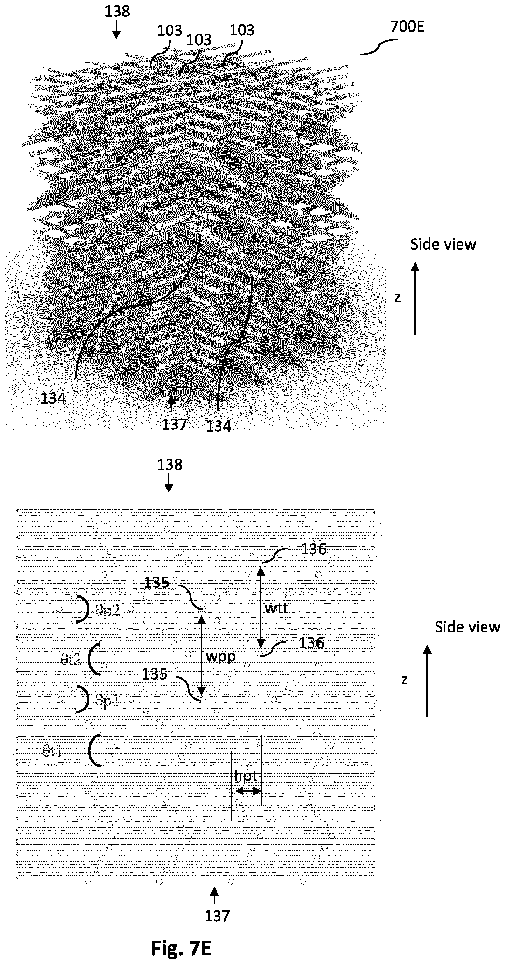

Fig. 7E shows an example of a further implant 700E. Fig. 7E includes a three-dimensional perspective cross-section (top image) and a two-dimensional cross-section (bottom image) of the implant 700E. Implant 700E may be similar to the implants 700A to 700D and may include one or more or all of the features of the implants.

As shown in the case of implant 700E, the undulating portion may include a zig-zag portion, wherein the angles θ (e.g. θt or θp) of the undulating portion may be different (or vary) from each other within the undulating portion of a sidewall 134. Varying the angle θ may change the softness of the implant. For example, portions of the implant with smaller θ may be softer than portions of the implant with larger θ. Additionally or optionally, features such as the peak-to-trough height, hpt, peak-to-peak width, wpp, and/or trough-to-trough width, wtt, may vary (differ from each other) within the undulating portion of the sidewall 134.

It may be understood that the features of Figs. 7A to 7G may be combined with each other to form an implant having the desired softness and elasticity.

An (or each) undulating sidewall may include at least one of a zig-zag portion and a sinusoidal portion. Optionally, the said zig-zag (or sinusoidal) portion may extend over (or may include) the entire length of the channel 103. Alternatively, the said zig-zag (or sinusoidal) portion may extend over (or may include) a selected portion (e.g. proportion, or fraction, or segment) of the entire length of the channel 103. In some examples, one or more or all of these parameters (angles θt, θp, hpt, wpp, wtt) may be constant (e.g. the same) throughout (or within) the entire undulating portion. In other examples, these parameters may vary throughout (or within) the entire undulating portion. In some examples, the undulating portion may include the entirety (e.g. the whole) length of the sidewall 134. In some examples, the undulating portion may include between 10 % and 90 % (or e.g. between 20 % and 80 %, or e.g. between 30 % and 70 %) of the entire sidewall 134. For example, the sidewall may include an undulating portion and a straight portion. In some examples, the sidewall 134 may include any number and/or combination of zig-zag portions, sinusoidal portions and straight portions.

The sidewalls 134 of a channel 103 (e.g. the sidewalls enclosing a channel 103) may be similar (same or e.g. identical) to each other. Alternatively, or optionally, they may be dependent on each other (e.g. being a mirror-image of each other). Alternatively, or optionally, they may be different from each other. In some examples, all the sidewalls of an implant may be sinusoidal sidewalls. In other examples, all the sidewalls of the implant may be zig-zag sidewalls. In some examples, the sidewalls of the implant may be a mixture of zig-zag and sinusoidal sidewalls.

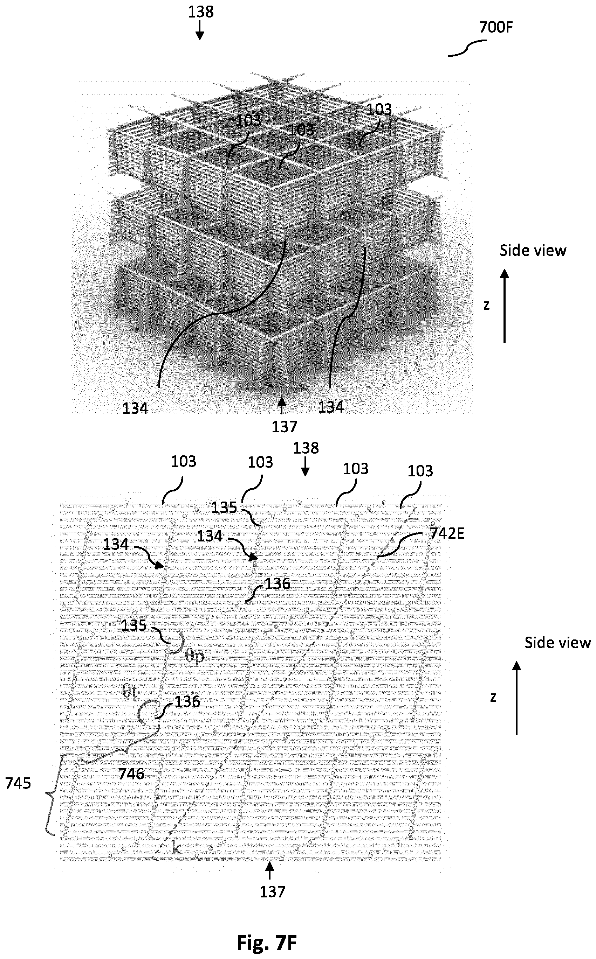

Fig. 7F shows an example of a further implant 700F. Fig. 7F includes a three-dimensional perspective cross-section (top image) and a two-dimensional cross-section (bottom image) of the implant 700F. Implant 700F may be similar to the implants 700A to 700E and may include one or more or all of the features of the implants.

As shown in the Fig. 7F, the implant 700F may include tilted, zig-zag (sinusoidal) channels, whereas Figs. 7A to 7E showed channels extending parallel to the z-direction.

In tilted channels, an acute tilt angle, k, between a longitudinal axis 742E and a reference axis (e.g. an x-axis or y-axis) representing the first outer surface region may be less than 90 degrees, or for example, less than 60 degrees.

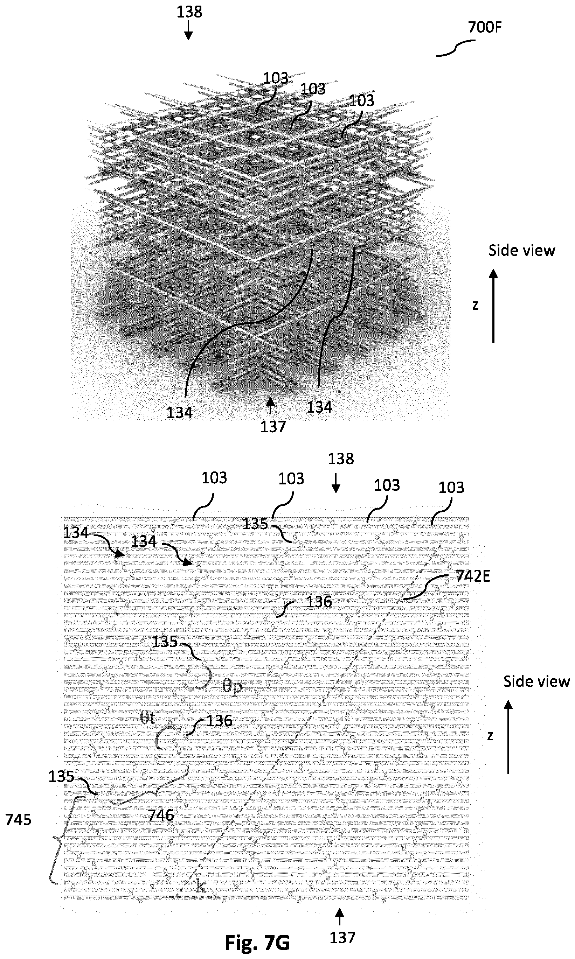

Fig. 7G shows an example of a further implant 700G. Fig. 7G includes a three-dimensional perspective cross-section (top image) and a two-dimensional cross-section (bottom image) of the implant 700G. Implant 700G may be similar to the implants 700A to 700F and may include one or more or all of the features of the implants.

Fig. 7G shows a the sidewalls having a fractal zig-zag portion (e.g. zig-zagging portions within a zig-zagging portion). In other words, first plurality of consecutive strands 745 instead of forming a smooth surface, may include zig-zagging portions. Additionally or optionally, the second plurality of consecutive strands 746, instead of forming a smooth surface or line, may include zig-zagging portions.

In the implants of Figs. 7A to 7G, a vertical height peak-to-trough height, hpt, between a peak and an adjacent neighboring trough, and a peak-to-peak width, wpp, (and/or trough-to-trough width, wtt) between directly consecutive peaks may be between 1 % and 99 % (or e.g. between 1 % and 50 %, or e.g. between 1 % and 20 %, or e.g. between 1 % and 5 %) of a maximum dimension of the implant. The maximum dimension of the implant may be the largest dimension of the implant in the x-direction, y, direction or z-direction.

**Claims**

1. A three-dimensional implant (100, 200, 300) for tissue reconstruction or tissue augmentation for insertion into a patient, the implant comprising:

   a plurality of planar layers, wherein a first group of sublayers (115) comprises a plurality of strands oriented in first direction, wherein a second group of sublayers (116) comprises a plurality of strands oriented in a second direction, the sublayers of the first group of sublayers (115) and the sublayers of the second group of sublayers (116) are arranged alternatingly in a third direction, the plurality of layers forming a three-dimensional structure comprising a plurality of hollow channels (103) extending in the third direction, wherein the implant is compressible at least along the third direction;
   wherein each hollow channel (103) comprises a first sidewall (104A) extending in the third direction and comprises a plurality of strand segments (105A) oriented in a first direction and a plurality of gaps (106A) arranged alternatingly, and a second sidewall (104B) extending in the third direction and comprising a plurality of strand segments (105B) oriented in the second direction and a plurality of gaps (106B) arranged alternatingly, wherein at least one of the first sidewall (104A) and the second sidewall (104B) of the hollow channel is an undulating sidewall,
   wherein the plurality of strand segments (105A, 105B) of the undulating sidewall (104A, 104B) belong to different layers of the implant, wherein adjacent strand segments (105A, 105B) of the undulating sidewall (104A, 104B) are separated by a gap (106), wherein the adjacent strand segments (105A, 105B) have a lateral offset with respect to each other to create a pattern of a plurality of peaks (135) and a plurality of troughs (136) of the undulating sidewall (104A, 104B).

2. The implant of claim 1, wherein the undulating sidewall (104A, 104B) comprises at least one of a zig-zag portion and a sinusoidal portion.

**Patentansprüche**

1. Dreidimensionales Implantat (100, 200, 300) zur Geweberekonstruktion oder Gewebevergrößerung zum Einsetzen in einen Patienten, wobei das Implantat aufweist:

   eine Vielzahl von planaren Schichten, wobei eine erste Gruppe von Teilschichten (115) eine Vielzahl von Strängen aufweist, die in einer ersten Richtung ausgerichtet sind, wobei eine zweite Gruppe von Teilschichten (116) eine Vielzahl von Strängen aufweist, die in einer zweiten Richtung ausgerichtet sind, wobei die Teilschichten der ersten Gruppe von Teilschichten (115) und die Teilschichten der zweiten Gruppe von Teilschichten (116) abwechselnd in einer dritten Richtung angeordnet sind, wobei die Vielzahl von Schichten eine dreidimensionale Struktur bildet, die eine Vielzahl von hohlen Kanälen (103) aufweist, die sich in der dritten Richtung erstrecken, wobei das Implantat zumindest entlang der dritten Richtung komprimierbar ist;
   wobei jeder Hohlkanal (103) eine erste Seitenwand (104A) aufweist, die sich in der dritten Richtung erstreckt und eine Vielzahl von Strangsegmenten (105A), die in einer ersten Richtung ausgerichtet sind, und eine Vielzahl von Lücken (106A) aufweist, die abwechselnd angeordnet sind, und eine zweite Seitenwand (104B), die sich in der dritten Richtung erstreckt und eine Vielzahl von Strangsegmenten (105B), die in der zweiten Richtung ausgerichtet sind, und eine Vielzahl von Lücken (106B) aufweist, die abwechselnd angeordnet sind, wobei mindestens die erste Seitenwand (104A) und/oder die zweite Seitenwand (104B) des Hohlkanals eine wellenförmige Seitenwand ist,
   wobei die Vielzahl von Strangsegmenten (105A, 105B) der wellenförmigen Seitenwand (104A, 104B) zu verschiedenen Schichten des Implantats gehören, wobei benachbarte Strangsegmente (105A, 105B) der wellenförmigen Seitenwand (104A, 104B) durch eine Lücke (106) getrennt sind, wobei die benachbarten Strangsegmente (105A, 105B) einen seitlichen Versatz zueinander aufweisen, um ein Muster aus einer Vielzahl von Spitzen (135) und einer Vielzahl von Mulden (136) der wellenförmigen Seitenwand (104A, 104B) zu erzeugen.

2. Implantat nach Anspruch 2, wobei die wellenförmige Seitenwand (104A, 104B) mindestens einen Zick-Zack-Abschnitt oder einen sinusförmigen Abschnitt aufweist.

**Revendications**

1. Implant tridimensionnel (100, 200, 300) pour la reconstruction ou l'augmentation des tissus à insérer dans un patient, l'implant comprenant :

   une pluralité de couches planes, dans lesquelles un premier groupe de sous-couches (115) comprend une pluralité de brins orientés dans une première direction, dans lesquelles un deuxième groupe de sous-couches (116) comprend une pluralité de brins orientés dans une deuxième direction, les sous-couches du premier groupe de sous-couches (115) et les sous-couches du deuxième groupe de sous-couches (116) sont disposées alternativement dans une troisième direction, la pluralité de couches formant une structure tridimensionnelle comprenant une pluralité de canaux creux (103) s'étendant dans la troisième direction, dans laquelle l'implant est compressible au moins le long de la troisième direction;
   dans lequel chaque canal creux (103) comprend une première paroi (104A) s'étendant dans la troisième direction et comprenant une pluralité de segments de brins (105A) orientés dans une première direction et une pluralité d'espaces (106A) disposés en alternance, et une deuxième paroi latérale (104B) s'étendant dans la troisième direction et comprenant une pluralité de segments de brins (105B) orientés dans la deuxième direction et une pluralité d'espaces (106B) disposés en alternance, dans laquelle au moins l'une de la première paroi latérale (104A) et de la deuxième paroi latérale (104B) du canal creux est une paroi latérale ondulée,
   dans lequel la pluralité de segments de brins (105A, 105B) de la paroi ondulée (104A, 104B) appartient à différentes couches de l'implant, dans lequel les segments de brins adjacents (105A, 105B) de la paroi ondulée (104A, 104B) sont séparés par un espace (106), dans lequel les segments de brins adjacents (105A, 105B) ont un décalage latéral les uns par rapport aux autres pour créer un motif de plusieurs pics (135) et de plusieurs creux (136) de la paroi latérale ondulée (104A, 104B).

2. L'implant de la revendication 2, dans lequel la paroi latérale ondulée (104A, 104B) comprend au moins une partie en zigzag et une partie sinusoïdale.

**Fig. 1A**

Channel
103
138 Sidewall 138
104B
105 106 105B 105A 106A

Channel
103A

100

Sidewall
104C

101

101

137 137
Sidewall 104 Sidewall 104A

z 107

y

**Fig. 1B**

100

105A

101

d

105B d

113
114

gh 106A

105C

101

101

105A

gl

z 107

y

**Fig. 1C**

**Fig. 1D**

**Fig. 1E**

**Fig. 1F**

**Fig. 1G**

105A

gh

105A

Offset = 0

**Fig. 1H**

z — 107

x

105A

gh    2 x lt

offset

105A

Offset > 0

**Fig. 1I**

z — 107

x

**Fig. 2A**

**Fig. 2B**

EP 4 185 238 B1

**Fig. 3A**

Fig. 3B

Fig. 3C

**Fig. 3D**

**Fig. 4A**

**Fig. 4B**

**Fig. 4C**

**Fig. 4D**

**Fig. 5A**

**Fig. 5B**

**Fig. 5C**

**Fig. 5D**

| Determining parameters |
| Forming a plurality of strands |

**Fig. 6**

Fig. 7A

138
103 103 103
101 700B

Side view

z

134
137 134

103 138 103 103

134
742B

$\theta p$ $\theta p$
135 135
135
136
$\theta t$ $\theta t$
135
746
135
745 136

137

**Fig. 7B**

**Fig. 7C**

**Fig. 7D**

138    103  103    103                    700E

134

137  134

138

136

135
θp2        wtt
θt2    wpp    136
135
θp1

θt1
hpt

Side view
z

Side view
z

137

**Fig. 7E**

**Fig. 7F**

**Fig. 7G**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 20186961
- EP 2995278 A1
- US 20140243995 A1
- US 10004585 B